(19)

**Europäisches Patentamt**
**European Patent Office**
**Office européen des brevets**

(11) **EP 4 000 728 B1**

(12) **EUROPEAN PATENT SPECIFICATION**

(45) Date of publication and mention
of the grant of the patent:
**29.03.2023 Bulletin 2023/13**

(21) Application number: **20383003.9**

(22) Date of filing: **18.11.2020**

(51) International Patent Classification (IPC):
**B01J 27/18** (2006.01)    **B01J 35/00** (2006.01)
**C01B 25/32** (2006.01)    **B01J 37/08** (2006.01)
**B01J 37/34** (2006.01)    **C07C 227/12** (2006.01)
**C07C 29/36** (2006.01)    **C07C 227/00** (2006.01)

(52) Cooperative Patent Classification (CPC):
(C-Sets available)
**B01J 27/1806; B01J 35/002; B01J 35/004;
B01J 37/08; B01J 37/342; C07C 29/36;
C07C 227/00**        (Cont.)

(54) **COMPOSITION OR MATERIAL, A PROCESS FOR ITS PRODUCTION AND USES THEREOF**

ZUSAMMENSETZUNG ODER MATERIAL, VERFAHREN ZU SEINER HERSTELLUNG UND VERWENDUNGEN DAVON

COMPOSITION OU MATERIAL, PROCEDE POUR SA PRODUCTION ET SES UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.05.2022 Bulletin 2022/21**

(73) Proprietors:
• **B. Braun Surgical, S.A.**
  **08191 Rubi (Barcelona) (ES)**
• **Universitat Politècnica De Catalunya**
  **08034 Barcelona (ES)**

(72) Inventors:
• **ALEMÁN LLANSO, Carlos Enrique**
  **08019 Barcelona (ES)**
• **PUIGGALÍ BELLALTA, Jordi**
  **08019 Barcelona (ES)**
• **SANS, Jordi**
  **08019 Barcelona (ES)**
• **TURÓN DOLS, Pau**
  **08191 Rubí (Barcelona) (ES)**
• **SANZ BELTRÁN, Vanesa**
  **08191 Rubí (Barcelona) (ES)**

• **RODRÍGUEZ RIVERO, Anna Maria**
  **08191 Rubí (Barcelona) (ES)**

(74) Representative: **Patentanwälte
Ruff, Wilhelm, Beier, Dauster & Partner mbB
Kronenstraße 30
70174 Stuttgart (DE)**

(56) References cited:
**EP-A1- 3 278 818**

• **RIVAS MANUEL ET AL: "Sustainable synthesis
of amino acids by catalytic fixation of molecular
dinitrogen and carbon dioxide", GREEN
CHEMISTRY, vol. 20, no. 3, 18 December 2017
(2017-12-18), pages 685-693, XP055792877, GB
ISSN: 1463-9262, DOI: 10.1039/C7GC02911J**

**(Cont. next page)**

(52) Cooperative Patent Classification (CPC): (Cont.)

C-Sets
**C07C 29/36, C07C 31/08;**
**C07C 227/00, C07C 229/08**

**Description**

FIELD OF THE INVENTION

The present invention relates to a composition or material, a process for producing said composition or material and uses thereof.

BACKGROUND OF THE INVENTION

Synthetic mineral hydroxyapatite (HAp), a crystalline form of calcium phosphate with formula $Ca_{10}(PO_4)_6(OH)_2$, is of major biomedical interest because of its compositional and structural similarity with bones and teeth, which has motivated its use to repair and reconstruct such hard tissues. HAp has a hexagonal structure with space group $P6_3/m$ and cell dimensions a=b= 9.42 Å and c= 6.87 Å, which is stable up to 1273 °C.

The electrical properties of HAp were found to influence its biomedical application. Thus, there was an early interest in the polarization of HAp to generate a surface charge by applying a DC electric potential (i.e. from 1.0 to 10.0 kV/cm) at elevated temperatures, i.e. from 200 °C to 800 °C (Itoh, S.; Nakamura, S.; Kobayashi, T.; Shinomiya, K.; Yamashita, K.; Itoh, S. Effect of Electrical Polarization of Hydroxyapatite Ceramics on New Bone Formation. Calcif. Tissue Int. 2006, 78, 133-142).

Such thermally stimulated polarization (TSP) process causes defects inside crystal grains and originates space charge polarization in the grain boundaries, both inducing the formation of electrical dipoles (Nakamura, S.; Kobayashi, T.; Yamashita, K. Highly Orientated Calcification in Newly Formed Bones on Negatively Charged Hydroxyapatite Electrets. Key Eng. Mater. 2005, 284-286, 897-900).

However, the relaxation of such dipoles through time suggested that polarization was only partially maintained (semi-permanently), even though this effect was not quantified.

Recently, permanently polarized hydroxyapatite was synthesized by applying a constant DC voltage of 500 V (*i.e.* DC field of 3 kV/cm) at 1000 °C for 1 h to previously sintered crystalline HAp (cHAp) Rivas, M.; del Valle, L. J.; Armelin, E.; Bertran, O.; Turon, P.; Puiggali, J.; Alemán, C. Hydroxyapatite with Permanent Electrical Polarization: Preparation, Characterization, and Response against Inorganic Adsorbates. Chem. Phys. Chem. 2018, 19, 1746-1755). This TSP process caused important chemical changes, as the formation of OH⁻ defects (vacancies) and structural variations that resulted in an increment of the crystallinity. Consequently, the electrochemical properties and electrical conductivity of the resulting polarized mineral increased noticeably when compared with cHAp (i.e. sintered HAp without TSP treatment). For example, it was found that permanently polarized hydroxyapatite may be used as an electrophotocatalyst to obtain both glycine (Gly) and alanine (Ala; D/L racemic mixture) in mild reaction conditions (i.e. from atmospheric pressure to 6 bars and 95 °C) by fixing nitrogen from $N_2$ and carbon from $CO_2$ and $CH_4$ (Rivas, M.; del Valle, L. J.; Turon, P.; Alemán, C.; Puiggali, J. Sustainable Synthesis of Amino Acids by Catalytic Fixation of Molecular Dinitrogen and Carbon Dioxide. Green Chem. 2018, 20, 685-693). EP-A-3 278 828 discloses "permanently polarised hydroxyapatite" i.e. hydroxyapatite modified by exposing it to an electric field at high temperatures" and its use as a catalyst in the synthesis of amino acids.

Despite the progress already made, there remains further need for compositions or materials comprising permanently polarized hydroxyapatite, in particular as catalysts, preferably for synthesizing high valuable chemical products such as amino acids and other organic molecules.

OBJECT AND SOLUTION

In view of the foregoing, the object underlying the present invention is therefore to make available a composition or material, a process for its production and uses thereof which properly address the above-mentioned need.

This object is accomplished by a composition or material according to independent claim 1, a process for producing the composition or material according to claim 10 and uses of the composition or material according to claims 11 to 15. Preferred embodiments of the composition or material are defined in the dependent claims 2 to 9. Further preferred embodiments of the invention are defined in the present description. The subject-matter and wording, respectively of all claims is hereby incorporated into the description by explicit reference.

According to a first aspect, the present invention relates to a composition or material, in particular a catalytically active composition or material (i.e. a catalyst), comprising

- permanently polarized hydroxyapatite and
- brushite and/or a brushite-like material.

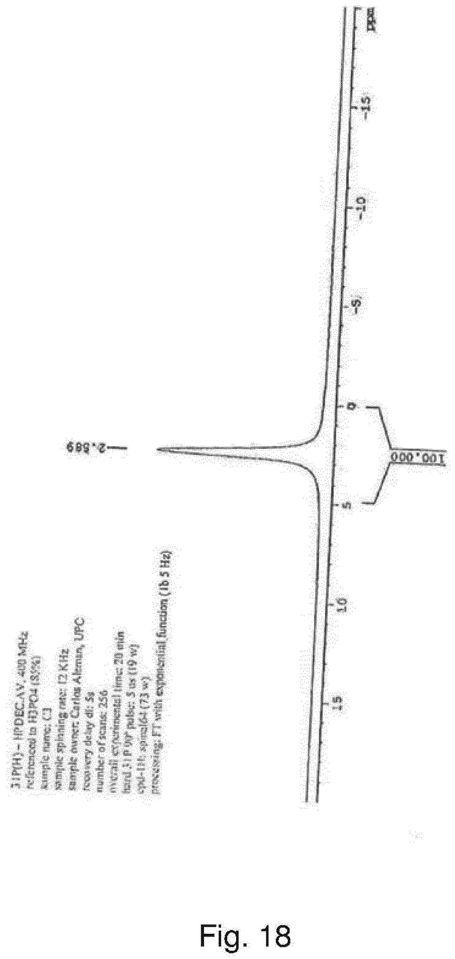

The term "permanently polarized hydroxyapatite" as used according to the present invention means a hydroxyapatite, in particular a synthetic hydroxyapatite, that has undergone a complete structural redistribution, in particular almost perfect, with a high crystallinity degree, i.e. particularly with a low amount of amorphous calcium phosphate and the presence of vacancies detected by increased electrochemical activity and the accumulation of charge per unit mass and surface. It has an electrochemical activity and ionic mobility which do not disappear over. The corresponding $^{31}$P-NMR spectrum of the permanently polarized hydroxyapatite is as shown on fig. 18. Said spectrum is carried out with solid hydroxyapatite using phosphoric acid ($H_3PO_4$) as a reference and showing a unique peak at 2.6 ppm corresponding to phosphate groups of hydroxyapatite.

The term "thermally polarized hydroxyapatite" as used according to the present invention preferably means a permanently polarized hydroxyapatite obtained or obtainable by a process (thermal polarization process) comprising the steps of

(a) sintering a sample of hydroxyapatite and/or amorphous calcium phosphate, in particular at a temperature between 700 °C and 1200 °C, and

(b) applying a constant or variable DC voltage, in particular between 250 V and 2500 V, in particular for at least 1 min at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a) or

applying an equivalent field, in particular between 1.49 kV/cm and 15 kV/cm, in particular for at least 1 min at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a) or

applying an electrostatic discharge, in particular between 2500 V and 1500000 V, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a) or

applying an equivalent electrical field, in particular between 148.9 kV/cm and 8928 kV/cm, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a).

The sample of hydroxyapatite in step (a) may be a natural, i.e. naturally occurring, hydroxyapatite or a synthetic hydroxyapatite.

Further, the sample of hydroxyapatite in step (a) may be in particular a sample of crystalline hydroxyapatite.

Accordingly, the permanently polarized hydroxyapatite of the composition or material according to the present invention is preferably obtained or obtainable by the above process (thermal polarization process).

The term "brushite" as used according to the present invention means a phosphate material or phosphate mineral, in particular synthetic phosphate material or synthetic phosphate mineral, with the chemical formula $CaHPO_4 \cdot 2H_2O$. In the WAXS (wide angle x-ray scattering) spectrum, the most representative peaks of brushite appear at $2\theta$ = 29°, 31°, 35°, 42°, and 51°, which have been attributed to the (141), (221 $\overline{)}$, (121), (152 $\overline{)}$ and (143 $\overline{)}$ reflections, respectively (JCPDS card number 72-0713).

The term "brushite-like material" as used according to the present invention refers to a calcium phosphate material that presents in the Raman spectrum peaks at 878, 848 and 794 cm$^{-1}$, which correspond to the normal vibration mode of $HPO_4^{2-}$, the POH deformation mode and the POH rotation mode, respectively.

The term "room temperature" as used according to the present invention means a temperature from 15 °C to 35 °C, in particular 18 °C to 30 °C, preferably 20 °C to 30 °C, more preferably 20 °C to 28 °C, particularly 20 °C to 25 °C.

The present invention rests on the surprising finding that a new and versatile composition or material, in particular in the form of a catalyst, can be in particular obtained by applying a thermally stimulated polarization (TSP) treatment to hydroxyapatite, wherein the hydroxyapatite is spaced from a positive electrode which together with a negative electrode is used for carrying out the TSP treatment. It surprisingly turned out that such a composition is in particular capable of producing or synthesizing high valuable organic molecules, in particular amino acids and/or other organic molecules such as carboxylic acids, aldehydes, ketones, alcohols and the like. Furthermore, it surprisingly turned out that the selectivity of reaction products, in particular with respect to their number of carbon atoms, may be advantageously tuned depending on the apparition of a brushite phase and/or brushite-like material phase, preferably on a surface of a permanently polarized hydroxyapatite phase, in particular on a surface of a permanently polarized hydroxyapatite main phase.

In an embodiment of the invention, the composition or material is in the form of a catalyst, preferably multi-phase, in particular bi-phase, catalyst, wherein the permanently polarized hydroxyapatite forms a phase, in particular a main phase, of the catalyst and the brushite and/or the brushite-like material form/forms a further phase of the catalyst.

The term "main phase" as used according to the present invention in the context of the composition or material, in particular being in the form of a catalyst, means a phase having a proportion, related to the total weight of the composition or material, in particular catalyst, which is larger than a proportion, also related to the total weight of the composition or material, in particular catalyst, of remaining phases or a remaining phase of the composition or material, in particular catalyst.

Preferably, the brushite and/or the brushite-like material is formed or present on a surface of the permanently polarized hydroxyapatite. In particular, the permanently polarized hydroxyapatite, to be more precise a surface thereof, is at least partially, in particular only partially or completely, layered or covered by the brushite and/or the brushite-like material.

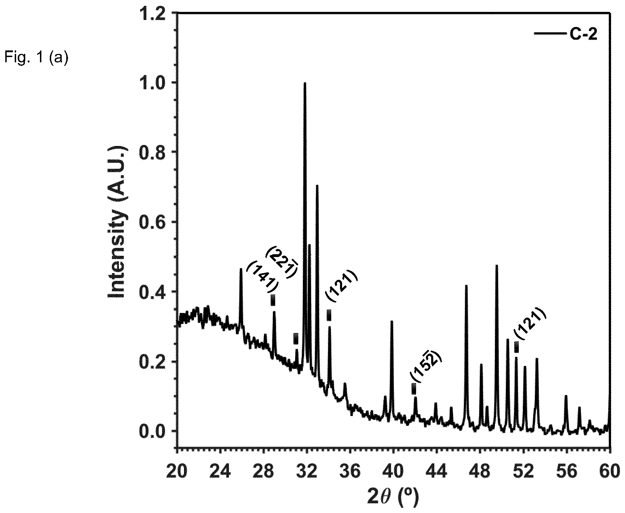

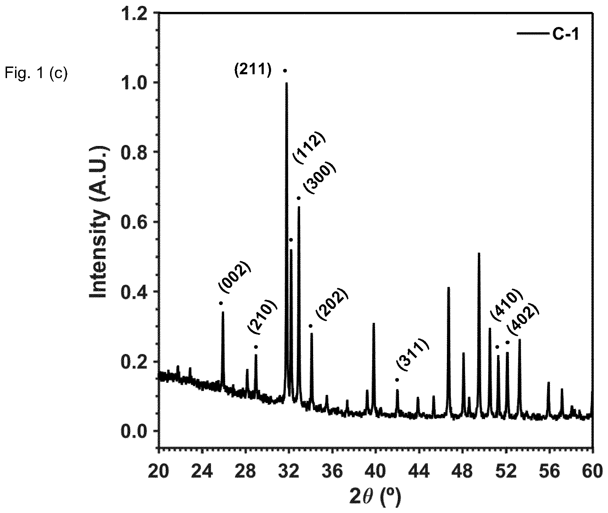

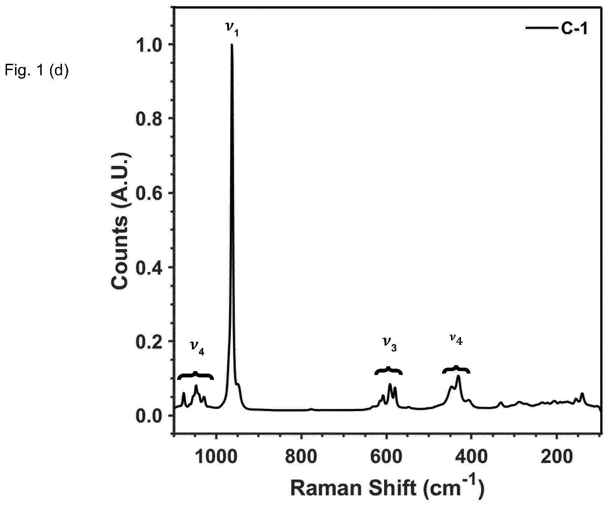

In a further embodiment, the composition or material has a wide angle x-ray scattering (WAXS) pattern as shown on fig. 1 (a). The x-ray scattering (WAXS) pattern shows peaks, in particular representative or unique peaks, at $2\theta = 29°$, $31°$, $35°$, $42°$, and $51°$, which have been attributed to the (141), (221̄), (121), (152̄) and (143̄) reflections, respectively (JCPDS card number 72-0713). Preferably, said pattern is carried out or obtained at room temperature, preferably at a temperature of 20 °C to 25 °C and/or under atmospheric conditions, in particular under atmospheric humidity and/or atmospheric pressure.

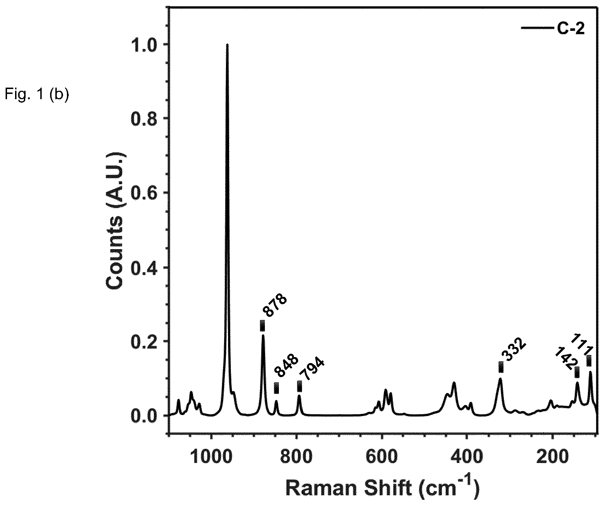

In a further embodiment, the composition or material has a Raman spectrum as shown on fig. 1(b). The Raman spectrum shows peaks at 878 cm$^{-1}$, 848 cm$^{-1}$ and 794 cm$^{-1}$. Said peaks correspond to the normal vibration mode of $HPO_4^{2-}$, the POH deformation mode and the POH rotation mode, respectively. Preferably, said spectrum is carried out or obtained at room temperature, preferably at a temperature of 20 °C to 25 °C and/or under atmospheric conditions, in particular under atmospheric humidity and/or atmospheric pressure, in particular recorded using a laser with a wavelength at 532 nm.

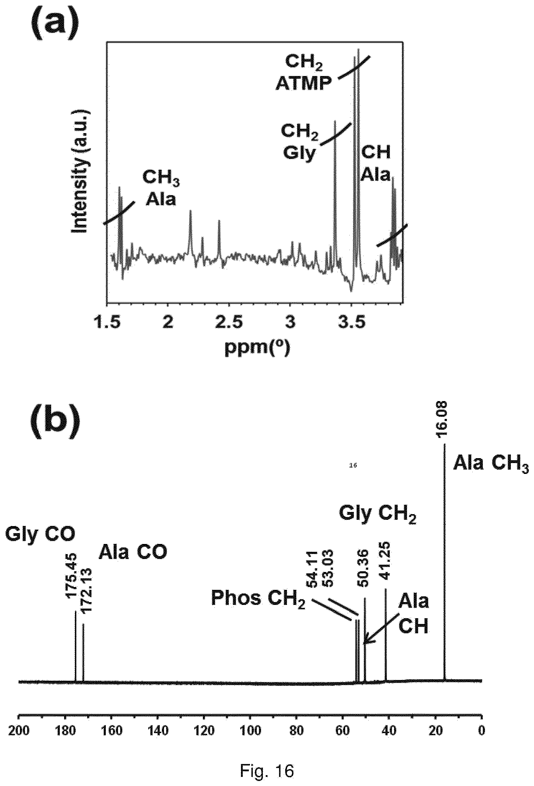

Further, the permanently polarized hydroxyapatite has a $^{31}$P-NMR spectrum as shown on figure 16. Said spectrum shows a unique peak at 2.6 ppm or around 2.6 ppm, i.e. in the range of 2.5 ppm to 2.7 ppm, corresponding to phosphate groups of hydroxyapatite. Said spectrum is carried out or obtained with solid permanently polarized hydroxyapatite at a temperature of 20 °C to 25 °C and using phosphoric acid ($H_3PO_4$) as a reference.

Further, the permanently polarized hydroxyapatite may in particular have a crystallinity, in particular determined by means of wide angle x-ray scattering (WAXS), from $\geq$ 65%, in particular 65 % to 99.9 %, preferably 75 % to 99 %, more preferably 80 % to 95 %.

Further, crystallites of the permanently polarized hydroxyapatite may in particular have a size, in particular determined by means of wide angle x-ray scattering (WAXS), from 20 nm to 500 nm, in particular 50 nm to 200 nm, preferably 70 nm to 100 nm. Preferably, in this context, the term "size" refers to an average diameter of the crystallites of the permanently polarized hydroxyapatite.

With respect to further features and advantages of the permanently polarized hydroxyapatite as used according to the present invention, it is referred to the PCT application WO 2018/024727 A1.

The permanently polarized hydroxyapatite has a proportion of 50 % by weight to 99.9 % by weight, in particular 65 % by weight to 99.9 % by weight, in particular 75 % by weight to 99 % by weight, preferably 80 % by weight to 95 % by weight, in particular 85 % by weight to 90 % by weight, in particular 85 % by weight to 88 % by weight, based on the total weight of the composition or material.

In a further embodiment of the invention, the brushite and/or the brushite-like material has a crystallinity, in particular determined by means of wide angle x-ray scattering (WAXS), from 65 % to 99.9 %, in particular 75 % to 99 %, preferably 80 % to 95 %.

In a further embodiment of the invention, crystallites of the brushite and/or the brushite-like material has a size, in particular determined by means of wide angle x-ray scattering (WAXS), from 20 nm to 500 nm, in particular 50 nm to 200 nm, preferably 70 nm to 100 nm. Preferably, in this context, the term "size" refers to an average diameter of the crystallites of the brushite and/or the brushite-like material.

In a further embodiment of the invention, the brushite and/or the brushite-like material has a proportion of > 0 % by weight to 50 % by weight, in particular 0.1 % by weight to 35 % by weight, in particular 1 % by weight to 25 % by weight, preferably 5 % by weight to 20 % by weight, in particular 10 % by weight to 15 % by weight, in particular 12 % by weight to 15 % by weight, based on the total weight of the composition or material.

Further, the composition or material may in particular have a total catalytic activity ratio of the permanently polarized hydroxyapatite to the brushite and/or the brushite-like material of 0.5 : 2, in particular 0.75 : 1.5, preferably 0.8 : 1.25, with respect to the sum of yield of all products of a product mixture. The total catalytic activity may be preferably determined by means of $^1$H-NMR (nuclear magnetic resonance) spectroscopy. For that purpose, the areas of the peaks in the $^1$H-NMR spectrum are preferably normalized according to the number of the protons for each product obtained.

Further, the composition or material may have a proportion of amorphous calcium phosphate of > 0 % by weight to 15 % by weight, in particular from > 0 % by weight to 10 % by weight, preferably > 0 % by weight to 5 % by weight, based on the total weight of the composition or material.

Further, the composition or material may be free of amorphous calcium phosphate.

Further, the composition or material may have a proportion of tricalcium phosphate, in particular β-tricalcium phosphate, of > 0 % by weight to 15 % by weight, in particular > 0 % by weight to 10 % by weight, preferably > 0 % by weight to 5 % by weight, based on the total weight of the composition or material.

Further, the composition or material may be free of tricalcium phosphate, in particular β-tricalcium phosphate.

Further, the composition or material may have a bulk resistance of $10^7$ $\Omega$ cm$^2$ to $10^5$ $\Omega$ cm$^2$, in particular $10^7$ $\Omega$ cm$^2$ to $10^5$ $\Omega$ cm$^2$, preferably $10^5$ $\Omega$ cm$^2$. In particular, the bulk resistance may increase (only) from 4 % to 33 %, in particular 4 % to 63 %, preferably 4 % after 3 months. The term "bulk resistance" as used according to the present invention means resistance to the electron transfer and may be determined by means of electrochemical impedance spectroscopy.

Further, the composition or material may have a surface capacitance which decreases less than 15 %, in particular less than 8 %, after 3 months. Preferably, the composition or material may have a surface capacitance which decreases from 0 % or > 0 % to 15 %, more preferably from 0 % or > 0 % to 5 %, after 3 months. The term "surface capacitance" as used according to the present invention means capacitance attributed to surface changes of hydroxyapatite induced by a thermal polarization process and may be determined by means of electrochemical impedance spectroscopy.

Further, the composition or material may be in the form of particles. The particles may have a diameter, preferably mean diameter, in particular determined by means of wide angle x-ray scattering (WAXS), of 20 nm to 500 nm, in particular 50 nm to 200 nm, preferably 70 nm to 100 nm.

Further, the composition or material may be in the form of a powder, in particular having particles as mentioned in the preceding paragraph.

Further, the composition or material may be in the form of a shaped body. The shaped body may have a polygonal, for example triangular, quadratic or rectangular, pentagonal, hexagonal, heptagonal, octagonal or nonagonal, cross-section or a corner-less, in particular circular, oval-shaped or elliptical, cross-section.

In particular, the shaped body may be in the form of a disc, plate, cone (conus) or cylinder.

Further, the shaped body may have a thickness of > 0 cm to 10 cm, in particular > 0 cm to 1 cm, preferably > 0 cm to 0.2 cm.

Further, the composition or material may be in the form of a coating.

The composition or material may further comprise an active ingredient. The active ingredient may be in particular a biologically or pharmaceutically active ingredient. The active ingredient may be in particular selected from the group consisting of antimicrobial, more particularly antibiotic, ingredient, wound healing-promoting ingredient, disinfecting ingredient, antiinflammatory ingredient, blood coagulation-promoting ingredient, growth factors, cell-differentiating factors, cell-adhesive factors, cell-recruiting factors, cell receptors, cell-binding factors, cytokines, peptides, structural proteins, extracellular proteins such as, for example, collagen, serum proteins such as, for example, albumin, polysaccharides such as, for example, hyaluronic acid, oligonucleotides, polynucleotides, DNA, RNA, salts thereof, stereoisomers, more particularly diastereomers, thereof and mixtures thereof.

For example, the active ingredient may be selected from the group consisting of biguanides, polyhexamethylene biguanide (PHMB), triclosan, chlorhexidine, gentamicin, vitamins, copper, zinc, silver, gold and mixtures thereof.

The composition or material may further comprise a material being selected from the group consisting of polymer, ceramic, silicate, organo-metallic compounds and mixtures thereof.

The polymer may be a biodegradable polymer, i.e. a polymer which degrades in vivo, i.e. within a human or animal body, or a non-biodegradable polymer. Further, the polymer may be a biopolymer, i.e. a naturally occurring polymer, or a synthetic, i.e. technical or not naturally occurring polymer. Further, the polymer may be a homopolymer or a copolymer, i.e. a polymer comprising at least two, in particular only two or more, different monomeric units.

The polymer may be in particular selected from the group consisting of polyolefines, polyesters, polyamides, polyimides, polyvinyl alcohols, polyurethanes, polycarbonates, polyalkylterephthalates, polyarylterephthalates, polyaryletherketones, polyhydroxyalkanoates, proteins such as extracellular proteins and/or globular proteins and/or enzymes and/or antibodies and/or blood clotting factors, polysaccharides and mixtures thereof.

In particular, the polymer may be selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, poylamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylene carbonate, poly-$\varepsilon$-caprolactone, collagen, gelatine, elastin, reticulin, fibronectin, laminin, fibrin, fibrinogen, albumin, starch, amylose, amylopectin, dextran, dextrin, cellulose, cellulose derivatives such as alkylcellulose, methylcellulose, hydroxyalkylcellulose, hydroxymethylcellulose, hydroxyethylcellulose, hydroxypropylcellulose, carboxyalkylcellulose, carboxymethylcellulose, chitin, chitosan, hyaluronic acid, dextran sulfate, heparin, heparan sulfate, chondroitin sulfate, dermatan sulfate, DNA, RNA, salts thereof, stereoisomers thereof, copolymers thereof and mixtures thereof.

The composition or material may further comprise an inorganic catalyst, in particular an inorganic photocatalyst. More specifically, the composition or material may be least partially, in particular only partially or completely, coated with an inorganic catalyst, in particular photocatalyst. The inorganic catalyst may be a catalyst such as $TiO_2$, $MgO_2$, $MnO_2$ or combinations thereof.

Further, the composition or material may be least partially, in particular only partially or completely, coated with aminotris(methylenephosphonic acid) and/or zirconium oxychloride ($ZrOCl_2$) and/or zirconia ($ZrO_2$). More specifically, the composition or material may have a three-layered coating, in particular wherein the three-layered coating may be composed of two layers of aminotris(methylenephosphonic acid) and a layer of zirconium oxychloride ($ZrOCl_2$) or zirconia ($ZrO_2$), wherein the layer of zirconium oxychloride is arranged or sandwiched between the two layers of aminotris(methylenephosphonic acid).

Further, the composition or material may be preferably a medical, in particular pharmaceutical, composition or material.

Further, the composition or material may be preferably a medical device, in particular an implant such as a bone implant or prosthesis, in particular knee or hip prosthesis.

According to a second aspect, the invention relates to a process for producing or synthesizing a composition or material according to the first aspect of the invention.

The process comprises the following steps:

(a) providing a sample of hydroxyapatite, in particular natural or synthetic hydroxyapatite, and/or amorphous calcium phosphate,

(b) sintering the sample of hydroxyapatite and/or amorphous calcium phosphate provided in step (a),

(c) applying a constant or variable DC voltage between 250 V and 2500 V, in particular for at least 1 min and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) or

applying an equivalent electric field between 1.49 kV/cm and 15 kV/cm, in particular for at least 1 min and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
applying an electrostatic discharge between 2500 V and 1500000 V, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
applying an equivalent electric field between 148.9 kV/cm and 8928 kV/cm, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) and

(d) cooling the sample obtained in step (c) maintaining the DC voltage or the equivalent electric field or

cooling the sample obtained in step (c) maintaining or without maintaining the electrostatic discharge or the equivalent electric field,
wherein, for performing step (c), the sintered sample of hydroxyapatite and/or amorphous calcium phosphate obtained in step (b) or the shaped body obtained from the sintered sample of hydroxyapatite and/or amorphous calcium phosphate obtained in step (b) is arranged between a positive electrode and a negative electrode, which are used for applying the constant or variable DC voltage, equivalent electric field or electrostatic discharge during step (c), such that the sintered sample of hydroxyapatite and/or amorphous calcium phosphate obtained in step (b) or the shaped body obtained from the sintered sample of hydroxyapatite and/or amorphous calcium phosphate obtained in step (b) is spaced from the positive electrode, i.e. such that the sintered sample of hydroxyapatite and/or amorphous calcium phosphate obtained in step (b) or the shaped body obtained from the sintered sample of hydroxyapatite and/or amorphous calcium phosphate obtained in step (b) has a distance to the positive electrode

For performing step (c), the sintered sample of hydroxyapatite and/or amorphous calcium phosphate obtained in step (b) or the shaped body obtained from the sintered sample of hydroxyapatite and/or amorphous calcium phosphate obtained in step (b) is arranged between the positive electrode and the negative electrode, which are used for applying the constant or variable DC voltage, equivalent electric field or electrostatic discharge during step (c), such that the sintered sample of hydroxyapatite and/or amorphous calcium phosphate obtained in step (b) or the shaped body obtained from the sintered sample of hydroxyapatite and/or amorphous calcium phosphate obtained in step (b) is spaced only from the positive electrode, i.e. such that the sintered sample of hydroxyapatite and/or amorphous calcium phosphate obtained in step (b) or the shaped body obtained from the sintered sample of hydroxyapatite and/or amorphous calcium phosphate obtained in step (b) has a distance only to the positive electrode. In other words, the sintered sample of hydroxyapatite and/or amorphous calcium phosphate obtained in step (b) or the shaped body obtained from the sintered sample of hydroxyapatite and/or amorphous calcium phosphate obtained in step (b) is preferably left in contact with the other of the two electrodes, more preferably with the negative electrode.

Further, the sintered sample of hydroxyapatite and/or amorphous calcium phosphate obtained in step (b) or the shaped body obtained from the sintered sample of hydroxyapatite and/or amorphous calcium phosphate obtained in step (b) is spaced from the one of the two electrodes, preferably from the positive electrode, in a distance from > 0 cm to 10 cm, in particular > 0 cm to 5 cm, preferably > 0 cm to 0.1 cm.

The electrodes, in particular the positive electrode and/or the negative electrode, can be of different shapes. The electrodes, in particular the positive electrode and/or the negative electrode, may have a polygonal cross-section, for example triangular, quadratic or rectangular, pentagonal, hexagonal, heptagonal, octagonal or nonagonal cross-section, or a corner-less, in particular circular, oval-shaped or elliptical, cross-section. Further, the electrodes, in particular the positive electrode and/or the negative electrode, may be in the form of a plate or disc, cone (conus) or a cylinder.

Further, the electrodes, in particular the positive electrode and/or the negative electrode, can be made of steel, in particular stainless steel.

Further, the electrodes, i.e. the positive electrode and the negative electrode, may have a mutual distance from 0.01 mm to 10 cm, in particular 0.01 mm to 5 cm, preferably 0.01 mm to 1 mm.

Preferably, the step (a) comprises

- subjecting a suspension, in particular an aqueous-alcoholic suspension, containing calcium phosphate to a hydrothermal treatment.

The term "aqueous-alcoholic suspension" as used according to the present invention means a suspension containing water and an alcohol, in particular ethanol, as a solvent or solvent mixture.

Preferably, the suspension containing calcium phosphate is obtained by using an aqueous solution containing ammonium phosphate dibasic (diammonium hydrogen phosphate, $(NH_4)_2HPO_4$) and an alcoholic, in particular ethanolic, solution containing calcium nitride ($Ca(NO_3)_2$). More preferably, the pH of the calcium nitride is adjusted at 10 to 12, in particular 10 to 11.5, more preferably 10.5 to 11. More preferably, the aqueous solution containing ammonium phosphate dibasic is added to the alcoholic, in particular ethanolic, solution containing calcium nitride. Further, the resulting mixture is preferably stirred, in particular for > 0 h to 24 h, at room temperature to form the suspension containing calcium phosphate. The latter may also be termed as "aging" of the reaction mixture.

Further, the hydrothermal treatment is preferably carried out in an autoclave, in particular under a pressure of > 0 bar to 200 bar, in particular > 0 bar to 50 bar, preferably > 0 bar to 20 bar.

Further, the hydrothermal treatment is preferably carried out at a temperature of 50 °C to 240 °C, in particular 100 °C to 240 °C, more preferably 110 °C to 240 °C, more preferably 120 °C to 240 °C, more preferably 130 °C to 240 °C, more preferably 140 °C to 240 °C, especially preferably 150 °C to 240 °C.

Further, the hydrothermal treatment is preferably carried out for > 0 h to 48 h, in particular > 0 h to 24 h, preferably 12 h to 24 h.

More preferably, the hydrothermal treatment is carried out by autoclaving the afore-mentioned reaction mixture, in particular after aging, at 150 °C for 24 h.

The step (a) may further comprise

- cooling the hydrothermally treated suspension containing calcium phosphate to form a precipitate. In particular, the hydrothermally treated suspension containing calcium phosphate may be cooled to a temperature of 0 °C to 90 °C, in particular 10 °C to 75 °C, preferably of 25 °C.

The step (a) may further comprise

- separating the precipitate obtained by cooling the hydrothermally treated suspension containing calcium phosphate, in particular by means of centrifugation and/or filtration.

The step (a) may further comprise

- washing the separated precipitate obtained by cooling the hydrothermally treated suspension containing calcium phosphate, in particular using water and/or an alcohol, in particular ethanol.

The step (a) may further comprise

- freeze-drying the separated and optionally washed precipitate obtained by cooling the hydrothermally treated suspension containing calcium phosphate to produce hydroxyapatite, in particular synthetic hydroxyapatite. The freeze-drying may be carried out for 1 day to 4 days, in particular for 2 days to 3 days, preferably for 3 days.

Further, the sample of hydroxyapatite in step (a) may be in particular a sample of crystalline hydroxyapatite.

Further, the above-mentioned step (b) may be carried out at a temperature between 700 °C and 1150 °C, in particular between 800 °C and 1100 °C, in particular at 1000 °C. Further, the process according to the present invention preferably comprises between the step (b) and the step (c) a further step (bc)

- shaping, in particular pressing, the sintered sample of hydroxyapatite and/or amorphous calcium phosphate obtained in step (b) to form the shaped body.

Accordingly, the shaped body may be preferably obtained from the sintered sample of hydroxyapatite and/or amorphous calcium phosphate obtained in step (b) by shaping, in particular pressing, said sample.

In particular, the step (bc) may be carried out under a pressure of 1 MPa to 1000 MPa, in particular 100 MPa to 800 MPa, preferably 600 MPa to 700 MPa. Further, the step (bc) may be carried out for 1 min to 90 min, in particular 5 min to 50 min, preferably 10 min to 30 min.

The shaped body may have a polygonal, for example triangular, quadratic or rectangular, pentagonal, hexagonal, heptagonal, octagonal or nonagonal, or a corner-less, in particular circular, oval-shaped or elliptical, cross-section. Further, the shaped body may have a thickness of 0.1 cm to 10 cm, in particular 0.1 cm to 5 cm, preferably 0.5 cm to 2 cm.

Preferably, the shaped body is in the form of a disc, plate, cone (conus) or cylinder.

Further, the constant or variable DC voltage or the equivalent electric field may be applied in the above-mentioned step (c) for 1 h to 24 h, in particular 0.1 h to 10 h, in particular 1 h.

Further, the DC voltage applied in the above-mentioned step (c) is preferably 500 V, which is equivalent to a constant electric field of 3 kV/cm.

Further, the equivalent electric field applied in the above-mentioned step (c) is preferably 3 kV/cm.

Further, the temperature in step (c) is preferably at least 900 °C, more preferably at least 1000 °C, in particular 1000 °C. Preferably, the temperature in step (c) is 900 °C to 1200 °C, in particular 1000 °C to 1200°C.

Further, the above-mentioned step (d) may be carried out by cooling the sample obtained in step (c) to room temperature.

Further, the above-mentioned step (d) may be carried out for 1 min to 72 h, in particular 15 min to 5 h, preferably 15 min to 2 h.

Preferably, for performing the above-mentioned step (c), the sample obtained in step (b) or the shaped sample thereof is spaced > 0 cm to 10 cm, in particular 0.1 cm to 7 cm, preferably 1 cm to 5 cm, from the one of the two electrodes, preferably from the positive electrode. Preferably, the sample obtained in step (b) or the shaped sample thereof is left in physical contact with the other of the two electrodes, preferably with the negative electrode.

Further preferably, the present invention relates to the use of the composition or material for the production or synthesis, in particular selective production or synthesis, of organic molecules, in particular functionalized organic molecules, i.e. organic molecules comprising or bearing a functional group, in particular selected from the group consisting of carboxylic group, amino group, aldehyde group (formyl group), keto group, hydroxy group and combinations thereof. The organic molecules, in particular functionalized organic molecules, preferably comprise or have 1 to 3 carbon atoms. Preferably, the organic molecules, in particular functionalized organic molecules, are selected from the group consisting of amino acids, carboxylic acids, aldehydes, ketones, alcohols and mixtures thereof. In particular, the amino acids/amino acid are/is glycine and/or alanine. Further, the carboxylic acids may be in particular selected from the group consisting of formic acid, acetic acid, malonic acid and mixtures thereof. Further, the ketone is preferably acetone. Further the alcohols/alcohol are/is preferably methanol and/or ethanol.

In a further embodiment of the invention, the present invention relates to the use of the composition or material as a catalyst, in particular electrocatalyst, preferably photoelectrocatalyst.

Preferably, said use as a catalyst is in a reaction for the production or synthesis, in particular selective production or synthesis, of organic molecules, in particular functionalized organic molecules, i.e. organic molecules comprising or bearing a functional group, in particular selected from the group consisting of carboxylic group, amino group, aldehyde group (formyl group), keto group, hydroxy group and combinations thereof. The organic molecules, in particular functionalized organic molecules, preferably comprise or have 1 to 3 carbon atoms.

In a further embodiment of the invention, said use as a catalyst is in a reaction for the production or synthesis, in particular selective production or synthesis, of amino acids, in particular natural amino acids, preferably glycine and/or alanine.

In a further embodiment of the invention, said use as a catalyst is in a reaction for the production or synthesis, in particular selective production or synthesis, of carboxylic acids, in particular formic acid, acetic acid, malonic acid or mixtures thereof.

In a further embodiment of the invention, said use as a catalyst is in a reaction for the production or synthesis, in particular selective production or synthesis, of aldehydes or ketones, in particular acetone.

In a further embodiment of the invention, said use as a catalyst is in a reaction for the production or synthesis, in particular selective production or synthesis, of alcohols, in particular methanol and/or ethanol.

Further, said use as a catalyst is preferably in a reaction for the production or synthesis of a mixture comprising or consisting of carboxylic acids, aldehydes, ketones, alcohols and mixtures of at least two of the afore-said organic molecules.

Further, said use as a catalyst is preferably in a reaction for the production or synthesis of a mixture comprising or consisting of ethanol and at least one further organic molecule, in particular selected from the group consisting of formic acid, acetic acid, malonic acid, acetone, methanol and a mixture of at least two of the afore-said organic molecules.

In particular, said use as a catalyst may be in a reaction for the production or synthesis of a mixture comprising or consisting of ethanol, formic acid, acetic acid, acetone and methanol.

In particular, said use as a catalyst may be in a reaction for the production or synthesis of a mixture comprising or consisting of ethanol, acetic acid, malonic acid, acetone and methanol.

In particular, said use as a catalyst may be in a reaction for the production or synthesis of a mixture comprising or consisting of ethanol, acetic acid and acetone.

Further, the present invention in particular relates to the use of the composition or material in biomedical applications. Preferably, said biomedical application is selected from the group consisting of cementum for teeth, bone, prosthesis, medical devices, drug-delivery, gene therapy and tissue regeneration.

Further, the present invention in particular relates to the use of the composition or material as electrodes.

Further, the present invention in particular relates to the use of the composition or material for doping polymers.

Further, the present invention in particular relates to the use of the composition or material for supporting, preferably adsorbing, organic molecules. Preferably, the organic molecules are selected from the group consisting of organo-metallic compounds, carbohydrates, amino acids, lipids, ATP, polymers and combinations, in particular mixtures, thereof. As regards said polymers, reference is made in its entirety to the previous description.

Further, the present invention in particular relates to the use of the composition or material for supporting, preferably adsorbing, phosphorous containing compounds such as pyrophosphate, triphosphate, triphosphonate, polyphosphates or combinations, in particular mixtures, thereof. The polyphosphates may be selected from any of the polyphosphates having from 1 to 50000 monomer units or any combination thereof.

Further, the present invention in particular relates to the use of the composition or material for supporting, preferably adsorbing, organo-metallic compounds, preferably metal phosphonates. The organo-metallic compounds are preferably compounds containing metal ions. Preferably, the metal ions are selected from the group consisting of transition metal ions, lanthanide ions and combinations thereof. More preferably, the organo-metallic compounds are compounds containing metal ions, wherein the metal ions are selected from the group consisting of $Sr^{2+}$, $Mg^{2+}$, $Fe^{2+}$, $Fe^{3+}$, $Mn^{2+}$, $Zr^{2+}$, $Au^+$, $Ti^{4+}$ and combinations thereof.

Further, the present invention in particular relates to the use of the composition or material for molecular recognition, in particular racemic resolution.

Further, the present invention in particular relates to the composition or material for use in the treatment of bone degradation and/or bone malignancies, such as osteoporosis.

Further, the present invention in particular relates to the composition or material, in particular as a drug delivery system, for example for DNA and/or RNA.

Further, the present invention in particular relates to the composition or material for use in the prophylaxis and/or treatment of a disease. Preferably, the disease is selected from the group consisting of cancer, neuronal diseases and diseases related to tissue calcifications. More preferably, the disease is selected from the group consisting of genetic disorders including, but not limited thereto, achondroplasia, alpha-1 antitrypsin deficiency, antiphospholipid syndrome, autism, autosomal dominant polycystic kidney disease, breast cancer, charcot-marie-tooth, colon cancer, cri du chat, Crohn's disease, cystic fibrosis, dercum disease, Down syndrome, Duane syndrome, Duchenne muscular dystrophy, factor V Leiden thrombophilia, familial hypercholesterolemia, familial dediterranean fever, fragile X syndrome, Gaucher disease, hemochromatosis, hemophilia, holoprosencephaly, Huntington's disease, Klinefelter syndrome, Marfan syndrome, Myotonic dystrophy, neurofibromatosis, Noonan syndrome, osteogenesis imperfecta, Parkinson's disease, phenylketonuria, Poland anomaly, porphyria, progeria, prostate cancer, retinitis pigmentosa, severe combined immunodeficiency (SCID), sickle cell disease, skin cancer, spinal muscular atrophy, Tay-Sachs, thalassemia, trimethylaminuria, Turner syndrome, velocardiofacial vyndrome, WAGR syndrome, Wilson disease and diseases related to small and large arteries, heart valves, brain (where it is known as cranial calcification), joints and tendons, such as knee joints and rotator cuff tendons, soft tissues like breasts, muscles, and fat, kidney, bladder, and gallbladder.

Further, the present invention in particular relates to the use of the composition or material in a solid-state battery. As used herein, a solid-state battery is a battery that has both solid electrodes and solid electrolytes.

Further, the present invention in particular relates to the use of the composition or material in an energy harvesting chip which is a chip that can generate its own energy. Energy harvesting is defined as the conversion of ambient energy into usable electrical energy. When compared with the energy stored in common storage elements, like batteries and the like, the environment represents a relatively inexhaustible source of energy. Consequently, energy harvesting (i.e. scavenging) methods must be characterized by their power density rather than energy density.

Further, the present invention in particular relates to the use of the composition or material for removing harmful gases such as carbon dioxide, carbon monoxide, methane or mixtures thereof, in particular from the air or atmosphere.

With respect to further features and advantages of the uses described in the preceding paragraphs, reference is made in its entirety to the previous description.

Further features and advantages of the invention will become clear from the following figures, descriptions thereof and examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

BRIEF DESCRIPTION OF THE FIGURES

In the figures, the following is schematically displayed:

Figure 1. (a) (a) WAXS diffraction patterns in the characteristic $2\theta$ range from 20° to 60° for multiphasic HAp-brushite catalysts (hereafter named C-2); (b) Raman spectra of the $PO_4^{3-}$ internal modes ($v_1$, $v_2$, $v_3$ and $v_4$) for C-2. (c) WAXS diffraction patterns in the characteristic $2\theta$ range from 20° to 60° and (d) Raman spectra of the $PO_4^{3-}$ internal modes ($v_1$, $v_2$, $v_3$ and $v_4$) for single-phase hydroxyapatite (HAp) catalysts (hereafter named C-1) and C-2. Characteristic reflections and vibrations for HAp and brushite are labelled in each sub-figure.

Figure 2. Stacked Raman spectra obtained from a $7 \times 4$ array with a spacing of 1 $\mu$m for the C-1 and C-2 catalysts. Scale bar refers to the relative intensity of the peaks.

Figure 3. In-depth analyses of Raman spectra of C-1 and C-2 of the zones of interest: (a) v1 principal mode; (b) lattice modes; and (c) O-H stretching vibrational mode in the region of 3500 to 3700 $cm^{-1}$ (acquired with a 532 nm laser). Characteristic Raman vibrations for HAp and brushite are labelled in black and green, respectively.

Figure 4. XPS spectra of Ca 2p obtained for C-1 and C-2 catalysts.

Figure 5. 1H-NMR spectra of the regions at: (a) 0.5 to 4.0 ppm, (b) 5.5 to 8.5 ppm.

Figure 6. Comparison of catalytic performance of C-1 and C-2 catalysts: (a) ratio of products/ethanol, (b) C-2/C-1 ratio of all the products detected.

Figure 7. (a) Unit cell of HAp crystalline structure; (b) $OH^-$ channels along the c axis of HAp crystalline structure.

Figure 8. (a) Unit cell of brushite; (b) unit cell of monetite.

Figure 9. (a) and (b) Crystalline structure of brushite. The atoms involved in the hydrogen bonds have been labeled.

Figure 10. Raman spectra of (a) crystalline hydroxyapatite (hereafter named cHAp)($T_h$) and (b) thermally stimulated polarized crystalline (hereafter named cHAp/tsp)($T_h$) prepared using different hydrothermal temperatures for 24 h.

**Figure 11.** Raman spectra in the (a) 770-910 $cm^{-1}$ and (b) 100-350 $cm^{-1}$ intervals of cHAp(150 °C) and cHAp/tsp($T_h \geq$ 100 °C).

**Figure 12**. (a) Raman spectra of cHAp(150 °C) and cHAp/tsp(150 °C) prepared using different times for the HT: 10 h and 24 h. (b) Raman spectra of cHAp/tsp(150 °C) prepared by applying different DC voltages to cHAp(150 °C). (c) Raman spectra of cHAp/tsp(150 °) prepared by applying a voltage of 500 V and maintaining the steel electrodes in contact or not with the cHAp(150 °C) samples. cHAp(150 °C) were prepared applying the HT for 24 h in both (b) and (c).

**Figure 13**. (a) Raman spectra at different depths of cHAp/tsp(150 °C) obtained applying the HT for 24 h and a polarizing voltage of 500 V at 1000 °C. (b) Magnification of the spectra displayed in (a) for the 100-350 $cm^{-1}$ region.

**Figure 14.** Low magnification SEM micrographs of cHAp($T_h$) with $T_h$= 50, 100, 150, 200 and 240 °C. In all cases the HT was applied for 24 h. High magnification micrographs of cHAp(150 °C) are also displayed.

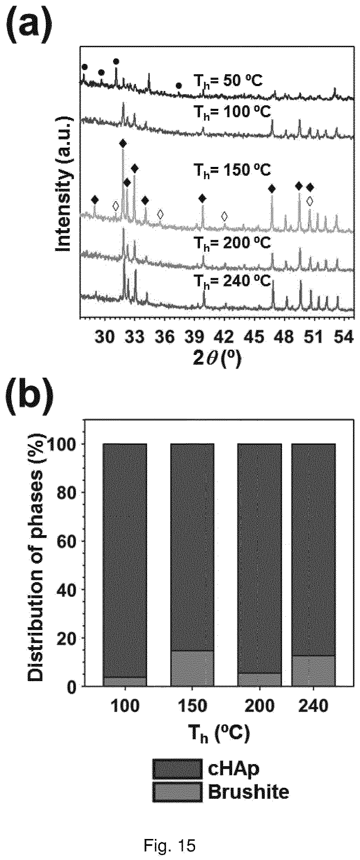

**Figure 15**. (a) X-ray diffraction patterns corresponding to cHAp($T_h$) with $T_h$= 50, 100, 150, 200 and 240 °C. In all cases the HT was applied for 24 h. Reflections attributed to cHAp and brushite are marked by filled diamonds and empty diamonds, respectively, in the diffractogram of cHAp(150 °C), while those associated with βTCP (β-tricalcium phosphate) are indicated by filled circles in the diffractogram of cHAp(50 °C). (b) Distribution of cHAp and brushite phases in cHAp($T_h$) with $T_h \geq$ 100 °C. This was obtained from the (211) reflection of cHAp and the (141) reflection of brushite.

**Figure 16.** For sample obtained after reaction (95 °C and 48 h) using a chamber pressure of 6 bar (i.e. 2 bar of each feeding reaction gas) and by the catalyst prepared using cHAp(150 °C): (a) $^1$H NMR spectrum of the solution obtained after extraction of the amino acids from the catalyst by dissolving the sample in deuterated water containing 100 mM of HCl and 50 mM of NaCl; and (b) solid state $^{13}$C NMR spectra of the catalyst with the synthesized amino acids.

**Figure 17.** Yield (in % per $cm^2$ of catalyst) of alanine and glycine in the electrophotocatalytic fixation of $N_2$, $CO_2$ and $CH_4$ as a function of $T_h$. In all cases reactions (in triplicate) were performed at 6 bar and 95 °C for 48 h.

**Figure 18**. 31P-NMR spectrum of permanently polarized hydroxyapatite.

EXPERIMENTAL SECTION

<u>1. Materials</u>

Calcium nitrate $Ca(NO_3)_2$, diammomium hydrogen phosphate [$(NH_4)_2HPO_4$; purity > 99.0%], ammonium hydroxide solution 30% [$NH_4OH$; purity: 28-30% w/w], zirconyl chloride (ZC; $ZrOCl_2 \cdot 8H_2O$) and aminotris(methylene phosphonic acid) (ATMP) were purchased from Sigma Aldrich. Ethanol (purity > 99.5%) was purchased from Scharlab. All experiments were performed with milli-Q water. $N_2$, $CH_4$ and $CO_2$ gases with a purity of >99.995% were purchased from Messer.

<u>2. Synthesis of crystalline hydroxyapatite (cHAp)</u>

15 mL of 500 mM $(NH_4)_2HPO_4$ in de-ionized water (pH adjusted to 10.5 $\pm$ 0.2 with ammonium hydroxide) were added drop-wise (2 mL/min) and under gentle agitation (100 rpm) to 25 mL of 500 mM $Ca(NO_3)_2$ in ethanol. The mixture was stirred for 1 h (100 rpm) at room temperature resulting in a suspension. Hydrothermal (HT) treatment was applied to the suspension with temperature ($T_h$) ranging from 50 °C to 240 °C using an autoclave Digestec DAB-2 for 24 h unless otherwise is specified. The autoclave was allowed to cool down before opening. The white precipitates were separated by centrifugation and washed sequentially at 8000 rpm for 5 minutes with water and a 60/40 v/v ethanol/water mixture (twice). After freeze-drying for 3 days the powder obtained was sintered at 1000 °C during 2 h at an air atmosphere, in particular using a Carbolite ELF11/6W/301 furnace. Hereafter, samples obtained using this procedure have been denoted cHAp($T_h$), where $T_h$ refers to the temperature used for the HT of hydroxyapatite (HAp).

### 3. Thermally stimulated polarization process (TSP)

According to a first approach, 150 mg of sintered cHAp powder were uniaxially pressed at 620 MPa for 10 minutes (i.e. 5 tons of applied weight) to obtain a disc of 10 mm of diameter and 1 mm of thickness. The disc was placed in between two stainless steel (AISI 304) plates separated at 4 cm and heated at 1000 °C in air atmosphere. Then, a DC voltage of 100 V, 500 V or 1000 V was applied during 1 h. Hereafter, samples obtained by applying the TSP process to $cHAp(T_h)$ are denoted $cHAp/tsp(T_h)$, the applied voltage being explicitly indicated in each case.

According to a second approach, catalytic activation was (also) successfully achieved applying the thermal stimulation polarization (TSP) treatment, which consisted in exposing the HAp disks at 500 V and 1000 °C for 1 h. For obtaining single-phase HAp catalysts (hereafter named C-1), the electrodes (two stainless steel AISI 304 plates) were placed in contact with the HAp disk. Instead, multiphasic HAp-brushite catalysts (hereafter named C-2) were attained by separating the positive electrode 4 cm from the HAp disk, which was left in contact with the negative electrode.

### 4. Characterization

Structural characterization studies were conducted using X-ray photoelectron spectroscopy (XPS), micro-Raman spectroscopy and wide angle X-ray scattering (WAXS). From WAXS spectra, crystallinity ($\chi_c$) and crystallite sizes $L_{hkl}$ were determined.

Further, the structural fingerprint of the samples was studied using the inVia Qontor confocal Raman microscope (Renishaw), equipped with a Renishaw Centrus 2957T2 detector. All measurements were performed with a 532 and a 785 nm laser. In order to achieve representative results, all the spectra presented in this study are the result of the average of a $105\times90$ $\mu$m grid with 42 points. Depth profiles were also obtained using the same equipment.

Morphological characterization has been performed by scanning electron microscopy (SEM) using a Focused Ion Beam Zeiss Neon40 microscope equipped with a SEM GEMINI column with a Shottky field emission. Samples were sputter-coated with a thin layer of carbon to prevent sample charging problems.

As mentioned, crystallinity ($\chi_c$) was obtained by wide angle X-ray scattering (WAXS) using a Brucker D8 Advance model with Bragg-Brentano $2\theta$ configuration and Cu $K_\alpha$ radiation ($\lambda = 0.1542$ nm). A one-dimensional Lynx Eye detector was employed. Measurements were performed in a $2\theta$ range of 20° - 60° in steps of 0.02°, and scan speed of 2 s. The $\chi_c$ value was obtained using the following expression:

$$\chi_c = 1 - \frac{V_{112/300}}{I_{300}} \qquad (1)$$

where $I_{300}$ is the intensity of the (300) reflection and $V_{112/300}$ is the intensity of the hollow between the (112) and (300) reflections. The crystallite size, $L_{hkl}$, was calculated using the Debye-Scherrer equation

$$L_{hkl} = \frac{0.9 \cdot \lambda}{B \cdot \cos\theta_{hkl}} \qquad (2)$$

where $\lambda$ is the wavelength of the monochromatic X-ray beam, $B$ is the full width at half maximum of the peak at the maximum intensity, and $\theta_{hkl}$ is the peak diffraction angle that satisfies the Bragg's law for the (hkl) plane.

### 5. Carbon Fixation

The reaction was carried out in an inert reactor chamber with a 3 bar $CO_2$ and 3 bar $CH_4$ atmosphere at 95 °C for 72 h under UV irradiation (GPH265T5L4, 253.7 nm). Before sealing, 0.5 mL of de-ionized liquid water were added to the reactor. The products of the reaction from the catalyst surface were analyzed by $^1$H-NMR spectroscopy (Bruker Avance III-400). Yields of the reaction were obtained using commercial products with controlled concentration as a reference.

### 6. Effect of TSP treatment on the coexistent brushite phase

Figure 1a, which compares the WAXS spectra recorded for C-1 and C-2, reveals the unambiguous presence of highly crystalline HAp in both catalysts. Thus, the peaks identified at $2\theta$ = 31.8°, 32.2°, and 33.0° correspond to the (211), (112) and (300) reflections of HAp, respectively (JCPDS card number 9-0432). The most significant peaks of the co-existent brushite appear at $2\theta = 29°$, $31°$, $35°$, $42°$, and $51°$, which have been attributed to the (141), (22$\overline{1}$), (121), (15$\overline{2}$) and (14$\overline{3}$) reflections, respectively (JCPDS card number 72-0713). As it can be seen, although the (22$\overline{1}$), $2\theta = 31.0°$, is the only distinguishable reflection for brushite in figure 1a, comparison of the relative intensities obtained for C-1 and C-2 supports the presence of this co-existing apatitic phase in the latter. One of the most relevant differences is observed at $2\theta = 51.3°$, which can be attributed to the (410) reflection of HAp or to the (14$\overline{3}$) of brushite. For C-1 the intensity of the peak at $2\theta = 51.3°$ relative to the one at $2\theta = 52.1°$, which corresponds to the (402) HAp reflection, is lower than one ($I_{2\theta=51.0} / I_{2\theta=52.1} = 0.96$), indicating that the intensity of the former peak is slightly lower than that of the latter. For C-2, $I_{2\theta=51.0} / I_{2\theta=52.1}$ increases to $I_{51/52.1} = 1.14$, evidencing that the peak at $2\theta = 51.3°$ becomes more intense than the one at $2\theta = 52.1°$. The (112) and (300) peaks were also used to determine the crystallinity ($\chi_c$; Eq S1), whereas the (211) reflection was used to calculate the crystallite size ($L_{211}$; Eq S2). The crystallinity is very high and similar for the two catalysts, $\chi_c = 0.95 \pm 0.03$ and $0.92 \pm 0.03$ for C1- and C-2, respectively. Crystallite sizes are also comparable for the two catalysts, the obtained values ($L_{211} = 75.2 \pm 2.4$ and $82.7 \pm 3.72$ nm for C-1 and C-2, respectively), being in agreement with those reported in the literature. Overall, these observations indicate that the differences applied during the TSP treatment do not affect the predominant HAp phase.

Raman studies on C-1 and C-2 catalysts are presented in figure 1b. The spectra of apatitic phases are mainly dominated by their characteristic P-O vibrations. The four characteristic regions of HAp, which correspond to the $PO_4^{3-}$ internal modes, can be seen in the spectra of the catalysts, being: $v_1 = 962$ cm$^{-1}$, $v_2 = 400\text{-}900$ cm$^{-1}$, $v_3 = 570\text{-}625$ cm$^{-1}$ and $v_4 = 1020\text{-}1095$ cm$^{-1}$. Both samples present a slight splitting of the $v_1$ mode in another two peaks at 970 and 949 cm$^{-1}$, which has attributed to the different P-O stretching vibrations of the three crystallographic non-equivalent $PO_4^{3-}$ tetrahedra found in the β-tricalcium phosphate (TCP) apatitic phase. Accordingly with the literature, the presence of TCP is not related to the TSP treatment but to small variation in the conditions during the HT synthesis and the sintering applied afterwards. Moreover, previous studies showed that the TSP treatment increases the crystallintiy and reduces TCP phase by imposing crystallographic specific orientations. Besides, the fact that the two samples present approximately the same relative amount of TCP (i.e. the ratio of the intensities of the Raman shift at 970 cm$^{-1}$ and the main peak at 962 cm$^{-1}$, $I_{970/962}$, is 0.12 and 0.10 for C-1 and C-2 respectively) evidences that differences in the conditions applied during the TSP treatment are not the precursors of the TCP generation. However, the effect of separating the positive electrode from the mineral disc during the TSP treatment is clearly manifested in figure 1b. More specifically, the presence of brushite in C-2 sample is confirmed by the peaks at 878, 848 and 794 cm$^{-1}$, which correspond to the normal vibration mode of $HPO_4^{2-}$, the POH deformation mode and the POH rotation mode, respectively. Other differences found between the Raman spectra of C-1 and C-2 catalysts are discussed below.

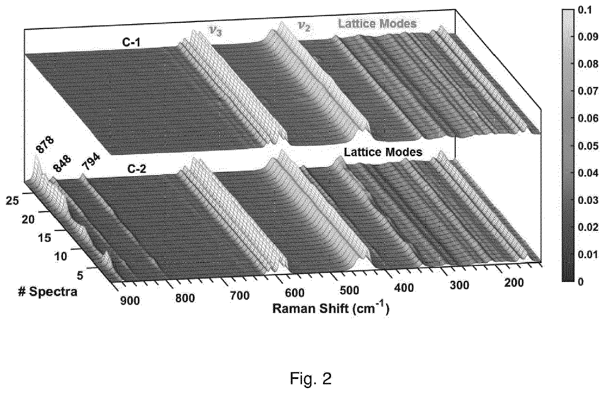

The evident presence of $HPO_4^{2-}$ and POH vibrations allow tracking the variations in the amount of brushite, depending on the TSP conditions. In this sense, the synergistic activity between the HAp and brushite phases is strongly dependent on their exposed surfaces and, therefore, characterization of their superficial distribution is of major interest. Figure 2 depicts 28 Raman spectra of C-1 and C-2 samples, which have recorded from a $7 \times 4$ array with a spacing of 1 $\mu$m. For clarity purposes, all spectra have been stacked together, the scale bar referring to the relative intensity of the peaks. As expected, the C-1 spectra do not show any trace of the brushite peaks aforementioned, in comparison with C-2 sample. Surprisingly, the 878 cm$^{-1}$ peak of C-2, which corresponds to the $HPO_4^{2-}$ normal vibration mode, presents variation in its relative intensity up to 90%, indicating that the coexisting brushite phase is distributed very heterogeneously. Results derived from figure 2 suggest that the geometry of the set-up used to apply the electric field plays an important role, defining the heterogeneity of the two co-existing phases. In order to support this conclusion, the inventors conducted additional experiments repeating the TSP treatment with the separated positive electrode. More specifically, the plate was removed, leaving only the cooper cable acting as the positive electrode. Examination of the recorded Raman spectra confirmed the dependence of the brushite phase with the geometry of the applied field. Not only the peaks $HPO_4^{2-}$ and POH vibration peaks appeared more intense (i.e. $I_{878/962} = 0.22$ and $I_{878/962} = 0.73$ for the C-2 catalysts prepared without and with plate at the positive electrode, respectively), but also the sample was more homogenous, the peak at 878 cm$^{-1}$ presenting a relative variation of ~21 % only. Overall, results demonstrate that the HAp/brushite ratio and heterogeneity of the catalyst can be regulated by varying the distance between the plates and the geometry of electric during the TSP treatment.

## 7. Structural Characterization of the C-2 catalyst

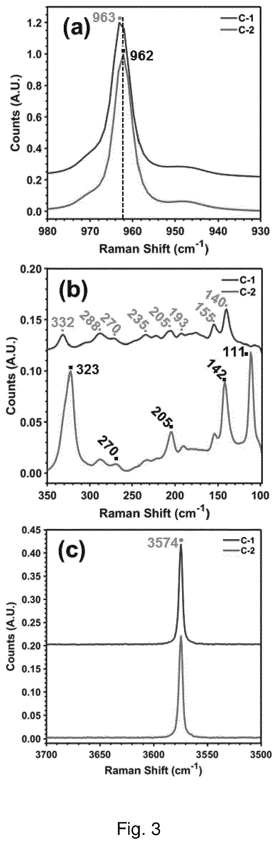

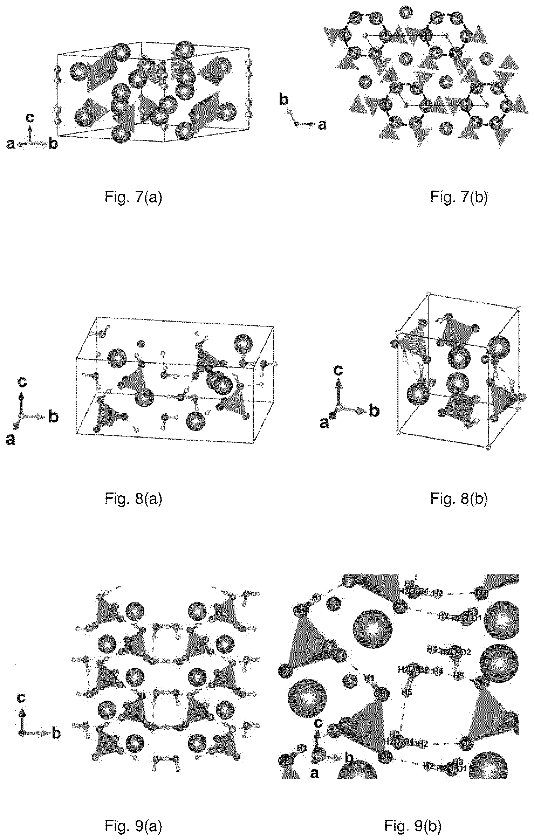

In order to understand the synergistic effects occurring in the brushite-containing catalyst, exhaustive structural characterization is crucial. In the case work, the inventor's efforts have been focused on discerning how brushite is integrated into the HAp crystal lattice as distortion on its boundaries, generating new active edge sites and increasing locally the electric conductivity. The most common polymorph of HAp is the hexagonal lattice (see figure 7) with the

space group $P6_3/m$ (a = b = 9.432 Å, c = 6.881 Å; $\alpha$ = $\beta$ = 90°, $\gamma$ = 120°) $PO_4^{3-}$ groups are ordered in equivalent tetrahedra while $Ca^{2+}$ ions occupy two different crystallographic positions. In this crystalline phase, the $OH^-$ groups are ordered in columns along the c-axis but with disordered orientations because of electrostatic forces. Upon application of the TSP treatment, $OH^-$ groups tend to orient along the specific direction of the electric field, thus introducing crystallographic stress in the crystal lattice. This crystallographic stress is shown for C-1 in figure 3a, which displays a magnification of the Raman spectrum in the region of the v1 principal mode. Whereas the main $v_1$ vibration mode of HAp of C-2 sample coincides with its theoretical value at 962 $cm^{-1}$, C-1 main peak is located at 963 $cm^{-1}$, reflecting the tensile strain of the $PO_4^{3-}$-tetrahedra. The fact that C-1 is the only sample presenting such shift indicates that the brushite found in the C-2 catalyst compensates the local tensile stress tension induced by re-arrangement of the $OH^-$ chains.

One of the most surprising aspects of the C-2 catalyst is that the attainment of the brushite phase is apparently contradictory with the fact that the TSP is carried out at high temperatures. At around 160 °C, brushite dehydrates to monetite ($CaHPO_4$) for further deprotonation to the $\gamma$, $\beta$ and $\alpha$ different forms of calcium pyrophosphate ($Ca_2P_2O_7$) when the temperature increases to 320, 700 and 1200 °C, respectively. The experimental evidence obtained from the $HPO_4^{2-}$ and the POH vibration modes at 878, 848 and 794 $cm^{-1}$, as well as the absence of the POP vibration at around 732 $cm^{-1}$, allow to discard the presence of $Ca_2P_2O_7$. The discrimination of brushite from monetite is clearly visualized by analyzing the lattice vibration modes since such structures crystallize in different space groups, as reflected in figure 8. Brushite consists on a monoclinic structure with an $Ia$ space group symmetry (a = 5.799 Å, b = 15.126 Å, c = 6.184 Å; $\alpha$ = $\gamma$ = 90°,

$\beta$ = 116.428°), while monetite exhibits a triclinic unit cell with a $P\overline{1}$ space group symmetry (a = 6.916 Å, b = 6.619 Å, c = 6.946 Å; $\alpha$ = 96.180°, $\beta$ = 103.82°, $\gamma$ = 88.34°). The lattice modes of the C-1 and C-2 catalysts are compared in figure 3b. C-1 presents the characteristic lattice modes of HAp at: 140 and 155 $cm^{-1}$ (attributed to the transitional vibrations of $Ca_1$ + $Ca_2$ and $Ca_2$, respectively); 193 and 205 $cm^{-1}$ (translational vibration of $PO_4^{3-}$);235 and 288 $cm^{-1}$ (librational vibrations of $PO_4^{3-}$);270 $cm^{-1}$ (transitional vibration of $Ca_1$); and 332 $cm^{-1}$ (translational vibrations of $OH^-$).

In comparison with C-1, the spectrum recorded for C-2 displays much more intense peaks at 111, 142 and 270 $cm^{-1}$, which have been attributed to the contribution of the $Ca^{2+}$ unique crystallographic sites in brushite. Moreover, the $PO_4^{3-}$ translational mode at 205 $cm^{-1}$ is enhanced in C-2 with respect to C-1. Is it worth noting that, while the peak at 142 $cm^{-1}$ (assigned to the transitional vibrations of $Ca_1+Ca_2$ of HAp and Ca of brushite) is much more intense and presents a red shift of 2 $cm^{-1}$ with respect to C-1, the peak at 155 $cm^{-1}$ remains unaltered, as it has been attributed to the HAp transitional vibration of $Ca_2$, which is inexistent in the brushite phase. The red shift of $Ca^{2+}$ transitional vibration from 140 $cm^{-1}$ in C-1 to 142 $cm^{-1}$ in C-2 confirms the presence of brushite in the latter, instead of monetite. Thus, due to group symmetries, the raman shift attributed to the $Ca^{2+}$ transitional vibration should be ordered as follows: monetite < HAp < brushite. Overall, the results shown in figure 3a-b not only confirms the presence of brushite but also highlights that the C-2 catalyst presents locally $Ca^{2+}$ with higher mobility and less tensile stress, which could cause a synergistic effect responsible of an enhance of its catalytic performance.

As shown in figure 9, the crystal structure of brushite can be understood as a layered system formed by zig-zag $Ca^{2+}$ and $PO_4^{3-}$ alternated chains parallel to the $a$-axis and growing along the c-axis. These chains are bond together along the b-axis through hydrogen bonds, provided by $H_2O$ molecules which form an intermediate layer. However, this water intermediate layer has not been detected in the Raman spectrum of C-2. Characteristic stretching modes for water should be detected in form of two duplets at 3539 and 3483 $cm^{-1}$ for $v_1$, and 3270 and 3163 $cm^{-1}$ for $v_2$. figure 3c shows the characteristic HAp peak corresponding to the $OH^-$ stretching mode of HAp at 3574 $cm^{-1}$. Moreover, neither the water liberations modes, which are typically located at 678 $cm^{-1}$, can be appreciated in figure 1b.

Detailed analysis of the lattice modes in figure 3b revealed the presence of a strong new peak at 323 $cm^{-1}$ for C-2, which has been assigned to the translational vibration modes of $OH^-$groups. This peak can be clearly deconvoluted into two different peaks located at 332 $cm^{-1}$, which matches the translational vibration of HAp obtained in the C-1 spectrum, and at 323 $cm^{-1}$. The fact that this new peak presents an important blue shift indicates the existence of a different crystallographic OH- with less mobility, and thus, somehow bonded. Due to their participation in hydrogen bonding interaction, $H_2O$ molecules of pure brushite are distorted from that of free molecules, generating two distinct crystallographic sites for water molecules (labeled in figure 9 as $H_2O$-O1 and $H_2O$-O2). The external layers are bond together via the O3···H2 (of $H_2O$-O1) and OH1...H4 (of $H_2O$-O2) hydrogen bond interactions, presenting short distances of 1.81 Å and 1.90 Å and almost linear angles of 167.3° and 175.7° respectively. Taking into account the theoretic aspects aforementioned and in agreement with the experimental results obtained, hydroxyl groups occupying the crystallographic positions of water molecules appear to be responsible for stabilizing the crystallographic brushite or brushite-like phase through hydrogen bonding interactions. The exchange of proton and hydroxyl groups at high temperatures, causing modifications in the crystalline structure of HAp, has been widely studied. However, substituting $H_2O$ molecules by OH- may cause other crystallographic distortions since the remaining water proton, which is hydrogen bonded to other oxygen of the lattice, is suppressed. For to case of $H_2O$-O2 water molecule this is not crucial for the stability of brushite, as H5 is weakly bonded to the $H_2O$-O1 water molecule, with a H5···$H_2O$-O1 distance of 2.16 Å. Accordingly,

the alternating $Ca^{2+}$ and $PO_4^{3-}$ ions form weaker interaction, and thus have more mobility. This hypothesis is supported by the drastic increment in the intensity of the lattice modes for such ions in the C-2 catalyst (figure 3b).

On the other hand, $H_2O$-O1 water molecule is also hydrogen bond to the O3 of a $PO_4^{3-}$ group, with a distance of 1.78 Å. In this case, determining which hydrogen bond is substituted by the $OH^-$ is harder, as they are energetically very similar. However, hypothesizing a combination of $OH^-$ ions pointing in both directions (O3···H2 and H3···O3) might be reasonable since the TSP treatment imposes a specific $OH^-$ orientation, whereas OH1···H4 and O3···H2 are pointing backwards.

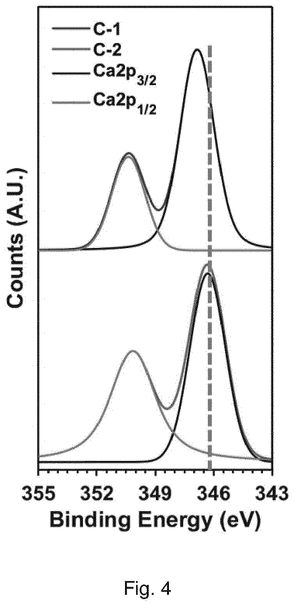

In order to quantify the enhancement in the lattice mobility of $Ca^{2+}$ ions highlighted by Raman spectroscopy, XPS measurements were conducted to capture the surface electron binding states of Ca. Figure 4 compares the XPS Ca2p of C-1 and C-2 catalysts. Both spectra present the characteristic Ca $2p_{3/2}$ and Ca $2p_{1/2}$ peaks of HAp located at 346.9 and 350.5 eV for C-1 and 346.4 and 350.0 eV for C-2. This observation is in good agreement with recent XPS studies on HAp after TSP treatment. The binding energy of the Ca 2p peak is mainly related with the Ca···$PO_4^{3-}$ bonds. The standard value of Ca $2p_{3/2}$ is usually measured at -347.2 eV, even though shifts at higher binding energies are observed in some cases, for example when $RCOO^-$ groups are adsorbed (i.e. Ca···$COO^-$ bonds are stronger than Ca···$PO_4^{3-}$ bonds). Instead, shifts to smaller binding energies are appreciated for C-1 and C-2, which has been associated to the $OH^-$ vacancies generated by the TSP treatment. In agreement with the Raman spectra, the shift is more pronounced for the C-2 sample (-0.8 eV) than for the C-1 sample (-0.3 eV). This 0.5 eV difference has been attributed to the presence of the brushite co-existent phase in the C-2 catalyst.

## 8. Effect of the presence of brushite on the selectivity of the catalyst

The exhaustive analysis accomplished in previous sections allowed discerning the structural differences between the C-1 and C-2 catalysts, highlighting some possible synergies between the two phases detected in the latter. More specifically, the effects of the coexisting brushite phase on the catalytic properties of HAp can be explained as follows: 1) $Ca^{2+}$ ions have smaller binding energy and, thus, can act as new catalytic or adsorption sites; and 2) even though the presence of $OH^-$ is maintained in both phases, the symmetry of ordered $OH^-$ columns may be broken in the boundaries between phases, creating regions with lower electron conductivity but with higher accumulated charge. In order to ascertain the possible synergies contributed by the brushite phase, carbon fixation reactions have been conducted using the C-1 and C-2 catalysts for comparison. More specifically, reactions have been catalyzed in an inert reaction chamber (120 mL) using a $CO_2$ and $CH_4$ gas mixture (3 bar each) and liquid water (1 mL) under irradiation of an UV lamp illuminating directly the catalysts at 95 °C.

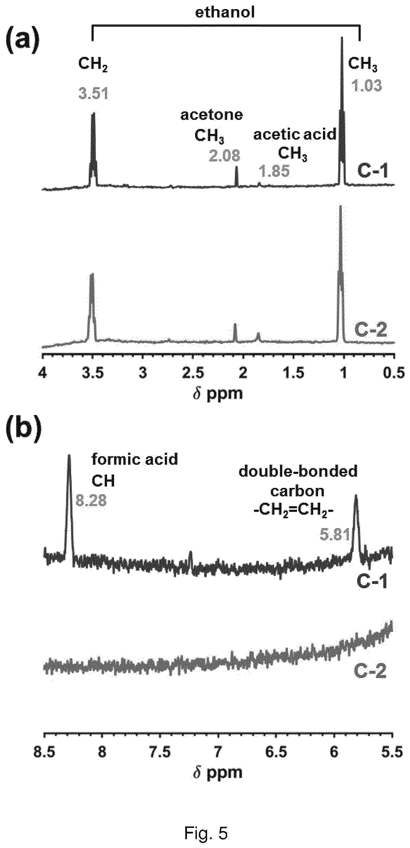

Figure 5 presents the $^1$H-NMR spectra obtained after 72 h reaction from the C-1 and C-2 catalysts dissolved in deuterated water containing 100 mM HCl and 50 mM NaCl, which allowed us to identify the reaction products formed on the surface of the catalysts. Three reaction products are clearly identified for both catalysts by inspecting the spectra at the low frequency region (figure 5a): ethanol ($CH_2$ quartet the $CH_3$ triplet at 3.50 and 1.06 ppm, respectively), acetone ($CH_3$ singlet at 2.08 ppm), and acetic acid ($CH_3$ singlet at 1.85 ppm). The OH peak of ethanol, which is the predominant product in both cases (i.e. 13.13 ± 3.75 and 15.01 ± 4.62 μmol per gram of catalyst for C-1 and C-2, respectively), overlaps the intense water peak at 4.65 ppm (not shown). In any case, the two spectra displayed in figure 5a are relatively similar, indicating that the coexistence of brushite does not play any significant effect on the yield of ethanol, acetone and acetic acid.

In contrast, analysis of the spectra $^1$H-NMR at the high frequency region (figure 5b) reveals a very important and noticeable difference. More specifically, formic acid (singlet at 8.28 ppm) and a peak at 5.81 ppm, which has been attributed to traces of double bonded carbon compounds are detected for C-1 sample only. This is an important achievement since the yield of formic acid, 8.06 ± 1.89 μmol, from C-1 is very high, reaching 36% of the total yields (vs. 59% for ethanol). Table 1 summarizes the yields of the reactions using C-1 and C-2:

Table 1. Yields (in μmol of product per gram of catalysts) for all the products of the reactions catalyzed by C-1 and C-2.

| Catalyst | Acetone | Acetic Acid | Ethanol | Formic Acid |
|----------|---------|-------------|---------|-------------|
| C-1 | 0.9±0.24 | 0.26±0.05 | 13.13±3.75 | 8.06±1.89 |
| C-2 | 1.31±0.29 | 0.65±0.13 | 15.01±4.62 | 0 |

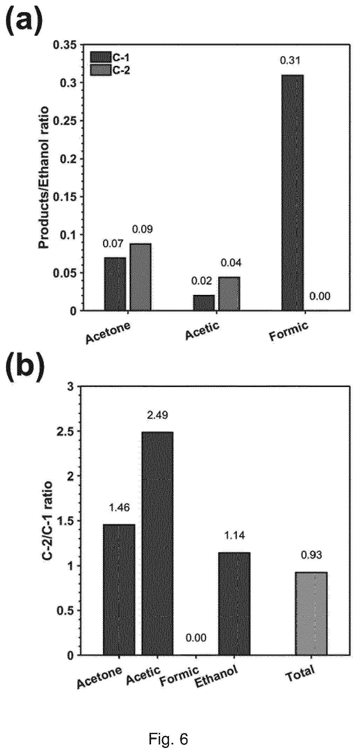

To further understand the selectivity differences between C-1 and C-2 catalysts, yields of the reaction with respect to ethanol production (figure 6a) and C-2/C-1 product ratios (figure 6b) have been analyzed. Firstly, the total catalytic activity ratio, which is defined by the C-2$_{total}$/C-1$_{total}$ ratio (where C-2$_{total}$ and C-1$_{total}$ refers to all the products obtained using the C-2 and C-1 catalysts, respectively), is 0.8. The fact that this value is close to 1 indicates that tuning the catalyst by introducing a new phase does not have any important effect on the total catalytic activity, but only the selectivity. Thus, the acetone, acetic acid and ethanol yields are higher for C-2 than for C-1, compensating the amount of formic acid generated. Furthermore, the results evidence that the coexistence of the HAp and brushite phases facilitates the incorporation of $\cdot CH_3$, favoring the conversion of formic acid to acetic acid is favored. Consistently, the yield of acetone is higher for C-2 than for C-1.

Overall, incorporation of small amounts of brushite phase in HAp-based catalysts enhances the incorporation of $\cdot CH_3$ species in $CO_2$ and $CH_4$ fixation reactions. This has been attributed to: 1) charge accumulation favors the dissociation of $CH_4$ to $\cdot CH_3$; and 2) $Ca^{2+}$ are more susceptible to adsorb species as they are less bonded.

9. Preparation of the cHAp/tsp-based catalyst

The 3-component catalyst was prepared by dropping successively 100 $\mu L$ of 50 mM ATMP, 10 mM ZC and 50 mM ATMP aqueous solutions on a cHAp/tsp disk (diameter: 10 mm; thickness: 1 mm). Before each dropping step, the sample was kept 8 h at room temperature for drying.

10. Synthesis of amino acids

A high pressure stainless steel reactor was employed to perform the synthesis of amino acids. The reactor was also characterized by an inert reaction chamber coated with a perfluorinated polymer (120 mL) where both the catalyst and water were incorporated. The reactor was equipped with an inlet valve for the entrance of $N_2$, $CH_4$, $CO_2$ and an outlet valve to recover the gaseous reaction products. A UV lamp (GPH265T5U4, 253.7 nm) was also placed in the middle of the reactor to irradiate the catalyst directly, the lamp being protected by a UV transparent quartz tube. All surfaces were coated with a thin film of a perfluorinated polymer in order to avoid any contact between the reaction medium and the reactor surfaces, in this way discarding other catalyst effects.

The reactions were performed at 95 °C for a reaction time of 48 h. Catalyst samples weighed approximately 150 mg and 0.5 mL of de-ionized liquid water were initially incorporated into the reaction chamber. The chamber was extensively purged with the first selected gas in order to eliminate the initial air content. Each selected gas was introduced to increase the reaction chamber pressure (measured at room temperature) to the target pressure. In all cases the chamber pressure was increased up to 6 bar by introducing sequentially 2 bar of each feeding reaction gas.

The reaction products were analyzed by NMR spectroscopy. All NMR spectra were acquired with a Bruker Avance III-400 spectrometer operating at frequencies of 400.1 and 100.6 MHz for $^1H$ and $^{13}C$, respectively. Chemical shifts were calibrated using tetramethylsilane ($^1H$ and $^{13}C$) as internal standard. Sixty-four and one thousand scans were recorded for $^1H$ and $^{13}C$ NMR, respectively. In order to remove the amino acids from the catalyst, samples were dissolved in deuterated water containing 100 mM of HCl and 50 mM of NaCl with the final addition of deuterated water.

11. cHAp: Temperature for the hydrothermal (HT) treatment

The precipitation and HT of HAp are crucial steps to adjust the stoichiometry and avoid the formation of other phases, such as $\beta$-tricalcium phosphate ($\beta$TCP) that can be easily formed depending on the conditions, and the sintering at 1000-1200 °C is frequently used to refine the crystal structure.

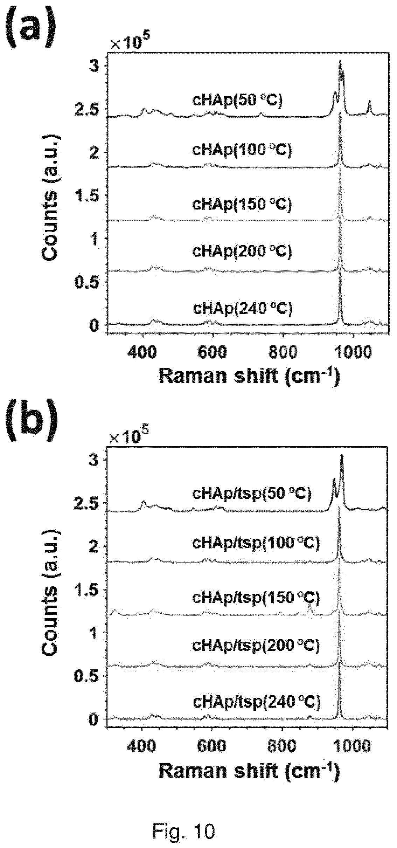

Figure 10a compares the Raman spectra recorded for cHAp($T_h$) samples prepared by applying, after the precipitation step, a HT at $T_h$= 50 °C, 100 °C, 150 °C, 200 °C and 240 °C for 24 h. The values of the normal-mode frequencies of the $PO_4^{3-}$ tetrahedron typically observed from Raman measurements in aqueous solution are $\upsilon_1$= 938 cm$^{-1}$, $\upsilon_2$= 420 cm$^{-1}$, $\upsilon_3$= 1017 cm$^{-1}$ and $\upsilon_4$= 567 cm$^{-1}$. In cHAp and $\beta$TCP, the crystalline field causes not only the shifts but also the splitting of the $PO_4^{3-}$ normal modes, even though these effects depend on the crystallographic structure. The spectra displayed in figure 10a clearly indicates that the splitting of the $PO_4^{3-}$ normal modes decreases with increasing hydrothermal temperature. For example, a single intense peak arising from the non-degenerate $\upsilon_1$ mode of $PO_4^{3-}$ at 962 cm$^{-1}$ is detected in the spectra of cHAp($T_h \geq$ 100 °C) samples, whereas several peaks can be observed for cHAp(50 °C). A similar feature is observed for the doubly degenerate $\upsilon_2$ and triply degenerate $\upsilon_3$ and $\upsilon_4$, which span a frequency range of 400-490, 570-625 and 1020-1095, respectively, for cHAp($T_h \geq$ 100 °C). In all cases the $PO_4^{3-}$ bands appear at frequencies 20-25 cm$^{-1}$ higher than those corresponding to the free-tetrahedral normal modes (i.e. in aqueous solution).

cHAp exhibits a $P6_3/m$ space group and the unit cell contains six equivalent $PO_4^{3-}$ tetrahedrons, whereas $\beta$TCP crystallizes in the R3c space group and its unit cell contains 42 $PO_4^{3-}$tetrahedrons distributed in three non-equivalent types. Consequently, the single intense peak detected at $\upsilon_1$= 962 cm$^{-1}$ is observed as two peaks and a shoulder for $\beta$TCP. This structural difference also affects $\upsilon_2$ and $\upsilon_4$, which span over a higher frequency range in $\beta$TCP than in cHAp. Moreover, $\upsilon_2$ and $\upsilon_4$ are separated by a frequency gap of only 55 cm$^{-1}$ in $\beta$TCP, while the gap is of 120 cm$^{-1}$ in cHAp. As it can be seen in Figure 10a, the fingerprints of the spectrum recorded for the cHAp(50 °C) sample agree with those expected for $\beta$TCP, indicating that this is the predominant phase when the HT is performed at low temperature.

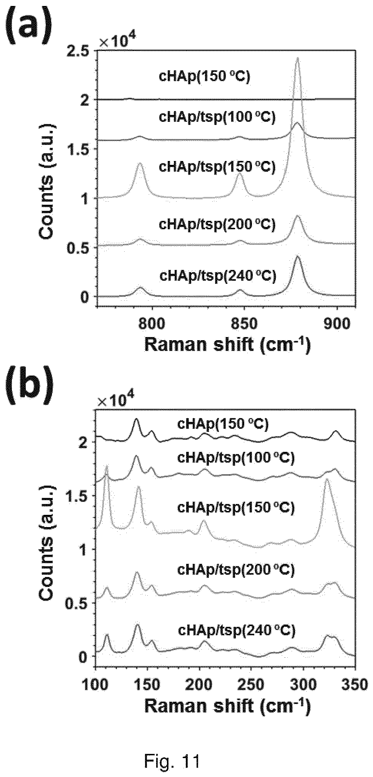

The Raman spectra obtained for cHAp/tsp($T_h$), which are displayed in figure 10b, show similar features to those

described for cHAp($T_h$) for the normal modes of $PO_4^{3-}$ (figure 10a). After the TSP treatment, the intensity and width of the peaks associated to the $PO_4^{3-}$ correspond to the βTCP phase for the samples prepared at $T_h$= 50 °C, while cHAp is clearly identified in samples hydrothermally treated at $T_h \geq$ 100 °C. However, some distinctive features can be also identified in cHAp/tsp($T_h$) samples as a function of $T_h$. For example, cHAp/tsp(150 °C) shows weak peaks at around 330 cm$^{-1}$, whereas no signal is detected for the rest of the samples. Considering that the detection of these peaks depend on the degree of crystallinity of the sample, Raman results suggest that the highest crystallinity is achieved when the HT is performed at $T_h$= 150 °C. Furthermore, a peak at 878 cm$^{-1}$ is detected for cHAp/tsp($T_h \geq$ 100 °C), as is clearly in the evidenced in figure 11a. This signal, which is characteristic of the Brushite mineral (i.e. $CaHPO_4 \cdot 2H_2O$) that is understood to be a precursor of apatite, has been attributed to the normal mode frequency of $HPO_4^{2-}$. The intensity of this band is much greater for the sample obtained at $T_h$= 150 °C, while for the rest it is observed as a weak peak. In addition, two bands typical of POH rotation and deformation modes appear at 794 and 848 cm$^{-1}$. Although they are very weak in all cases, POH signals also showed the highest intensity for samples obtained at $T_h$= 150 °C.

Figure 11b compares the weak intensity bands observed between 100 and 350 cm$^{-1}$ for cHAp(150 °C) and cHAp/tsp($T_h \geq$ 100 °C). These bands have been attributed to translational modes of the $Ca^{2+}$ (111, 139 and 154 cm$^{-1}$), $PO_4^{3-}$ (287 cm$^{-1}$) and OH- (331 and 323 cm$^{-1}$) sublattices, and rotational modes of the $PO_4^{3-}$ group (205 cm$^{-1}$). As it can be seen, both the intensity and narrowness of all these bands is maximum when the TSP process is applied to cHAp(150 °C) samples. Moreover, the intensity bands associated to the translational modes of the OH- sublattice yield (331 and 323 cm$^{-1}$ in figure 11b) increases linearly with that of the POH rotation and deformation modes (794 and 848 cm$^{-1}$ in figure 11a), evidencing a structural correlation.

## 12. Duration of the HT and characteristics of the TSP treatment

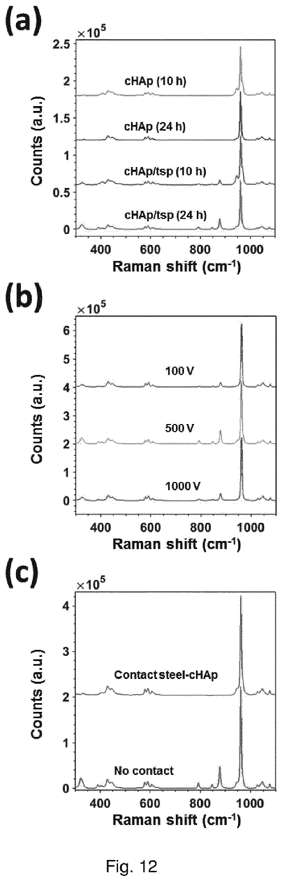

The duration of the HT is another factor that affects the structure of the HAp and, therefore, the performance of cHAp/tsp as catalyst. Figure 12a compares the Raman spectra recorded for samples prepared at $T_h$= 150 °C when the time for the HT was of 10 or 24 h. As it can be seen, the spectra of the cHAp samples obtained after a HT of only 10 h show the peaks described in the previous section for the βTCP phase. Thus, the conversion of the βTCP phase into cHAp is only completed when the HT is long enough, even when the $T_h$ was the optimum. Interestingly, figure 12a shows that the TSP treatment favors the re-arrangement of the surface in samples prepared using both 10 and 24 h, as is proved by the apparition of the Brushite peak at 878 cm$^{-1}$ and the translational modes of the OH- sublattice at 323 cm$^{-1}$. Nevertheless, the βTCP fingerprints identified for cHAp(150 °C) samples obtained using a treatment time of 10 h, which are associated to the four normal-mode frequencies of the $PO_4^{3-}$, remain practically unaltered after the permanent polarization process. Accordingly, the TSP process cannot be used to compensate the undesirable structural effects induced by the shortening of the HT.

The effect of the electric field strength used to induce permanent polarization in cHAp has been examined by applying DC voltages of 100 V, 500 V or 1000 V (25, 125, 250 V·cm$^{-1}$ respectively) to cHAp(150 °C) samples. Although the Raman spectra obtained for all the resulting samples correspond to cHAp (figure 12b), some bands apparently associated to the catalytic activity of cHAp/tsp are slightly influenced by the strength of the DC voltage. More specifically, the peaks attributed to translation modes of the OH- sublattice (323 cm$^{-1}$), the normal mode of $HPO_4^{2-}$ (878 cm$^{-1}$), and the POH rotation and deformation modes (794 and 848 cm$^{-1}$) are more intense and better defined for samples polarized at 500 V than for those obtained at 100 V and 1000 V. According to these results, the alteration of the voltage used for the TSP process is not expected to annihilate the activity of cHAp/tsp as catalyst but to induce small changes in its effectivity.

Finally, the influence of the geometry of the electrodes in the TSP treatment has been investigated. For this purpose, cHAp/tsp(150 °C) samples were prepared using a DC voltage of 500 V and two different geometries for the electrodes: i) steel plates separated at 4 cm and, therefore, the cHAp(150 °C) disc was in contact with one electrode only (i.e. the thickness of the sintered mineral discs was 1 mm); and *ii)* steel plates separated at 1 mm and, therefore, each side of the cHAp(150 °C) disc was in contact with an electrode. The recorded Raman spectra, which are compared in figure 12c, reveals that the bands at 794, 848 and 878 cm$^{-1}$ are only observed when the TSP process is conducted hindering the steel-cHAp contact. Also, the intensity of the band associated to the translational mode of the OH- sublattice (323 cm$^{-1}$) is much higher when the separation between the electrodes is of 4 cm.

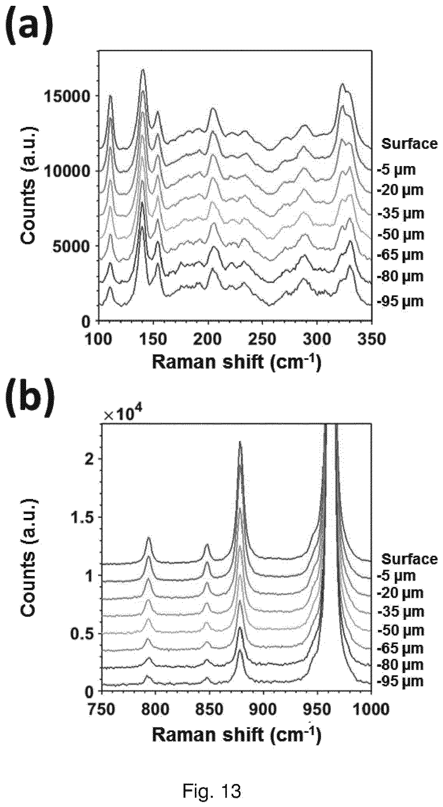

A depth profiling Raman analysis was conducted to monitor the extent of the changes induced by the TSP treatment. Figure 13a compares the Raman spectra recorded at different depths (i.e. from the surface to a depth of 95 $\mu$m) for cHAp/tsp(150 °C) prepared applying the HT for 24 h and using a polarizing voltage of 500 V at 1000 °C. Although the spectra show the same fingerprints in all cases, the intensity of the signals decreases with increasing depth. This observation is particularly remarkable for the bands of $HPO_4^{2-}$ (878 cm$^{-1}$) and both POH rotation and deformation modes (794 and 848 cm$^{-1}$). These results indicate that the changes caused by the TSP treatment are predominantly located at the surface of the mineral. This is corroborated in figure 13b, which displays the intrinsically weak bands detected in the 110 and 330 cm$^{-1}$ region. The intensity of the bands associated to the rotational modes of the $PO_4^{3-}$ group (205

$cm^{-1}$) and, specially, to the translational modes of the $Ca^{2+}$ (111, 139 and 154) $cm^{-1}$), $PO_4^{3-}$ (287 $cm^{-1}$) and OH- (323 $cm^{-1}$) sublattices decreases with increasing depth.

## 13. Morphological and structural characterization of cHAp samples

Raman spectra displayed in figures 10 and 11 indicate that the chemical properties of cHAp($T_h$) and cHAp/tsp($T_h$) depends on $T_h$ and, therefore, the catalytic activity of the latter. To investigate the implications of $T_h$ in the morphology and structure of cHAp, SEM and XRD have been used.

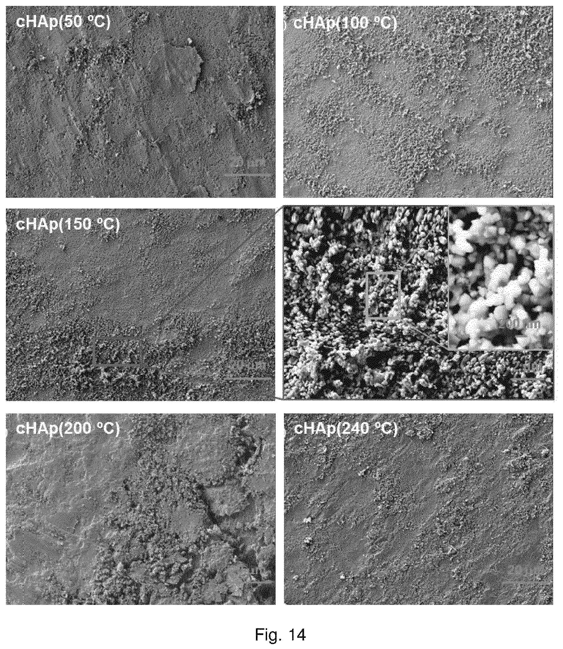

Low magnification SEM micrographs of cHAp($T_h$) with $T_h$= 50 °C, 100 °C, 150 °C, 200 °C and 240 °C are displayed in figure 14. With exception of the sample with βTCP ($T_h$= 50 °C), which presents the most compact surface, the rest of the samples presents a morphology made of compact regions alternated with extensive porous zones. Inspection of the high magnification micrographs, shown in figure 14 for cHAp(150 °C) as representative sample, indicate that porous zones are constituted by pillars that grow through the aggregation of mineral nanoparticles in a preferential direction. However, the morphology of the porous areas is apparently independent of $T_h$.

Structural characterization of prepared cHAp($T_h$) samples was completed by WAXD (Figure 15a). The characteristic fingerprint of hexagonal crystal symmetry cHAp (a= b= 9.421 Å, c= 6.881 Å, α= β= 90°, and γ= 120°; JCPDS card number 9-0432) is typically associated to the peaks at 32°-34° $2\theta$, which correspond to the (211), (112) and (300) reflections. These reflections are clearly identified in the diffraction profiles of all samples prepared using $T_h \geq$ 100 °C and can be intuited in that of the cHAp(50 °C) sample. Other characteristic reflection peaks of cHAp appear at $2\theta=$ 32°, 34°, 40°, 47° and 49°, which correspond to (211), (202) (130) (222) and (214) reflections. The (112) and (300) peaks were also used to determine the crystallinity ($\chi_c$: Eqn 1), whereas the (211) reflection was used to calculate the crystallite size ($L_{211}$; Eqn 2). The $\chi_c$ of cHAp($T_h$) is 0.53, 0.82, 0.68 and 0.77 for $T_h$= 100 °C, 150 °C, 200 °C and 240 °C, respectively, which is fully consistent with Raman spectra displayed in figure 10a. Thus, the $\chi_c$ of cHAp is maximum when it is prepared at $T_h$= 150 °C followed by that obtained at $T_h$= 240 °C. The variation detected in cHAp(200 °C) with a decrease in $\chi_c$ in relation to cHAp(150 °C) and cHAp(240 °C), and which is experimentally reproducible, has been attributed to combined effect of the lyophilization and sintering processes on the crystals, which seems to depend on the value of $T_h$ used. This could not only explain the reduction of $\chi_c$ when $T_h$ increases from 150 °C to 200 °C but also the fact that $\chi_c$ is lower for cHAp(240 °C) than for cHAp(150 °C). Besides, the calculated $L_{211}$ values correspond to 69.1, 82.7, 82.6 and 82.7 nm for cHAp($T_h$) with $T_h$= 100 °C, 150 °C, 200 °C and 240 °C, respectively.

On the other hand, the most relevant reflection peaks of brushite (JCPDS card number 72-0713), which has a monoclinic structure with cell parameters a= 5.812 Å, b= 15.180 Å, c= 6.239 Å, α=γ= 90° and β= 116.43°, are clearly observed in the X-ray diffraction pattern of cHAp(150 °C) (figure 15a). These reflections, which are weaker for cHAp(240 °C) and much weaker for cHAp(100 °C) and cHAp(200 °C), correspond to the (141), (121), (15$\bar{2}$) and (14$\bar{3}$) with $2\theta=$ 29°, 35°, 42° and 51°, respectively. It is worth mention that, although the position of some of these reflections, as for example the (14$\bar{3}$), matches other reflections found in the theoretical diffraction of pure cHAp, the changes in the relative intensities points to co-existence of the two phases, which is in agreement with Raman spectra. The amounts of cHAp and brushite phases in samples prepared at $T_h \geq$ 100 were roughly estimated using X-ray diffraction patterns by comparing the (211) reflection of cHAp and the (141) reflection of brushite. Results show that, although the formation of the latter phase is promoted when the HT is conducted at $T_h \geq$ 100 °C (figure 15b), cHAp is clearly the predominant phase in all cases. However, the content of Brushite increases from ~5% for $T_h$= 100 °C and 200 °C to -15% for $T_h$= 150 and 240 °C.

In general the anomalous behaviour of the samples obtained at $T_h$= 200 °C has been attributed to the dehydration process reported for Brushite at such temperature. Thus, the layered structure of Brushite, $CaHPO_4 \cdot 2H_2O$, in which mineral layers are held together by hydrogen bonded water molecules, converts into an amorphous phase and Monetite, $CaHPO_4$. More specifically, although surface water evaporates at around 100 °C, the two crystallographic water molecules of Brushite, which are associated by hydrogen bonds with oxygen atoms in phosphate group, remain stable at such temperature, leaving from the system at 200 °C. The structural transitions associated to the Brushite dehydration at 200 °C explains the reduction of the $\chi_c$, which in turn is in detriment of the catalytic activity.

Finally, inspection of the diffractogram obtained for cHAp(50 °C) (figure 15a) allows to recognize the reflection peaks typically reported for βTCP (JCPDS card number 09016), which predominate over those associated to the cHAp. Thus, the rhombohedral βTCP appears as the predominant crystalline phase in samples HT-treated at the lowest temperature.

## 14. Performance of cHAp/tsp($T_h$) as catalyst for the synthesis of amino acids

In a recent study, the inventors catalyzed the fixation of nitrogen from $N_2$ and carbon from $CO_2$ and $CH_4$ to obtain Gly (glycine) and Ala (alanine), the two simplest amino acids. The catalyst was prepared by coating cHAp/tsp(150 °C) samples with two layers of ATMP separated by an intermediate ZC layer. For this purpose, the cHAp/tsp(150 °C) disks were sequentially immersed in 5 mM ATMP, 5 mM ZC and 1.25 mM ATMP aqueous solutions at room temperature for 5 h. After each immersion, samples were dried at 37 °C for 3 h. The catalyzed reaction was conducted under UV light irradiation and mild reaction conditions, in an inert reaction chamber starting from a simple gas mixture containing $N_2$, $CO_2$, $CH_4$ and $H_2O$.

Figure 16 proves that change in the application of the ATMP and ZC coatings does not alter the performance of the catalyst. Figure 16a shows the $^1$H NMR spectrum of samples obtained by dissolving the catalyst prepared using cHAp(150 °C) and the products of reaction after 48 h at 95 °C. The signal corresponding to ATMP methylene group appears as doublet at 3.53-3.56 ppm, while the signals corresponding to the methylene group of produced Gly is singlet at 3.37 ppm and both methine and methyl groups of Ala are the quadruplet at 3.84-3.87 ppm and the doublet at 1.60-1.62 ppm, respectively. The same compounds are also detected in the $^{13}$C NMR spectrum displayed in figure 16b, where only peaks assigned to the ATMP (53.82 and 52.92 ppm), Gly (172.26 and 41.35 ppm) and Ala (175.26, 50.56 and 16.18 ppm) units are detected.

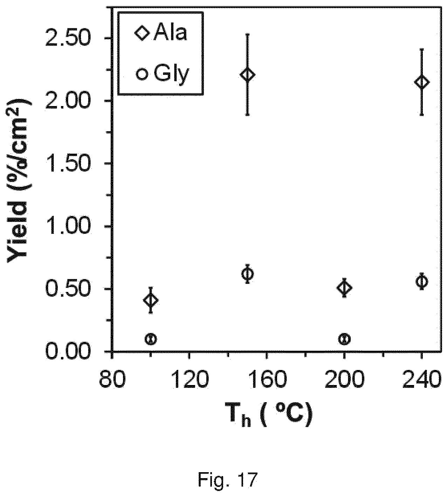

It should be noted that the reaction was positive also for catalysts prepared using cHAp($T_h$) with $T_h \geq 100°C$. The yield of the reaction was calculated using commercial Gly and Ala (purchased from Sigma-Aldrich) at a controlled concentration to calibrate the $^1$H NMR peaks. The variation of the yield of the reaction expressed in % per $cm^2$ of catalyst against $T_h$ is represented in Figure 17. These values are higher than those found in the inventor's previous study, which was 2.5 after 48 h (i.e. Gly/Ala ratio decreased from 5.4 to 2.2 when the reaction time increased from 2 to 96h). This feature has been attributed to the presence of the brushite phase, which was not observed in the catalyst prepared in the inventor's previous study. Accordingly, the combination of brushite and cHAp achieved with the process described in this work apparently accelerates the transformation of Gly into Ala.

On the other hand, the yield correlates with both the $\chi_c$ and the brushite content in the cHAp/tsp($T_h$). Thus, the maximum yield of amino acids after 48 h was obtained for the catalysts prepared at $T_h$= 150 °C and 240 °C (i.e. 2.8% and 2.7%, respectively), which displayed not only the highest $\chi_c$ (*i.e.* 0.82 and 0.77, respectively) but also the highest content of brushite (i.e. 15% and 12%, respectively). In contrast, the total yield decreases one order of magnitude, ~0.5-0.6%, for reactions catalyzed by cHAp/tsp($T_h$) samples with $\chi_c$ < 0.7. This feature clearly reflects the very important role of the spatially translations modes, which facilitates the transport of charge at the surface through oscillation and translational molecular movements upon excitation of the lattice.

## Claims

1.  Composition or material comprising

    - permanently polarized hydroxyapatite, wherein the corresponding $^{31}$P-NMR spectrum of the permanently polarized hydroxyapatite is carried out with solid hydroxyapatite using phosphoric acid as a reference and shows a unique peak at 2.6 ppm or around 2.6 ppm, i.e. in the range of 2.5 ppm to 2.7 ppm, and
    - brushite, wherein in the wide angle x-ray scattering spectrum the most representative peaks of brushite appear at $2\theta = 29°$, 31°, 35°, 42° and 51° and/or a brushite-like material, wherein the brushite-like material presents in the Raman spectrum peaks at 878, 848 and 794 $cm^{-1}$, and wherein the permanently polarized hydroxyapatite has a proportion of 50% by weight to 99.9% by weight, based on the total weight of the composition or material.

2.  The composition or material according to claim 1, **characterized in that** the composition or material is in the form of a catalyst, preferably multi-phase catalyst, wherein the permanently polarized hydroxyapatite forms a phase, in particular a main phase, of the catalyst and the brushite and/or brushite-like material forms a further phase of the catalyst.

3.  The composition or material according to claim 1 or 2, **characterized in that** the composition or material has a wide angle x-ray scattering pattern as shown on figure 1(a).

4.  The composition or material according to any of the preceding claims, **characterized in that** the composition or material has a Raman spectrum as shown on figure 1(b).

5.  The composition or material according to any of the preceding claims, **characterized in that** the permanently polarized hydroxyapatite has a proportion of 75 % by weight to 99 % by weight, preferably 80 % by weight to 95 % by weight, based on the total weight of the composition or material.

6.  The composition or material according to any of the preceding claims, **characterized in that** the brushite and/or the brushite-like material has a proportion of 0.1 % by weight to 35 % by weight, in particular 1 % by weight to 25 % by weight, preferably 5 % by weight to 20 % by weight, based on the total weight of the composition or material.

7.  Composition or material according to any of the preceding claims, **characterized in that** the brushite and/or the brushite-like material has a crystallinity, determined via wide angle x-ray scattering, from 65 % to 99.9 %, preferably 75 % to 99 %, more preferably 80 % to 95 %.

8.  Composition or material according to any of the preceding claims, **characterized in that** the brushite and/or the brushite-like material has a crystallite size, determined via wide angle x-ray scattering, from 20 nm to 500 nm, in particular 50 nm to 200 nm, preferably 70 nm to 100 nm.

9.  Process for producing a composition or material according to any of the preceding claims, **characterized in that** the process comprises the following steps:

    (a) providing a sample of hydroxyapatite and/or amorphous calcium phosphate,
    (b) sintering the sample of hydroxyapatite and/or amorphous calcium phosphate provided in step (a),
    (c) applying a constant or variable DC voltage between 250 V and 2500 V, in particular for at least 1 minute and/or at a temperature between 900 °C and 1200 °C, in particular from 1000 °C to 1200 °C, to the sintered sample of hydroxyapatite and/or amorphous calcium phosphate obtained in step (b) or to a shaped body obtained from the sintered sample of hydroxyapatite and/or amorphous calcium phosphate obtained in step (b) or

    applying an equivalent electric field between 1.49 kV/cm and 15 kV/cm, in particular for at least 1 minute and/or at a temperature between 900 °C and 1200 °C, in particular from 1000 °C to 1200 °C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
    applying an electrostatic discharge between 2500 V and 1500000 V, in particular for > 0 min to 24 h and/or at a temperature between 900 °C and 1200 °C, in particular from 1000 °C to 1200 °C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
    applying an equivalent electric field between 148.9 kV/cm and 8928 kV/cm, in particular for > 0 min to 24 h and/or at a temperature between 900 °C and 1200 °C, in particular from 1000 °C to 1200 °C, to the sintered sample of hydroxyapatite and/or amorphous calcium phosphate obtained in step (b) or to a shaped body obtained from the sintered sample of hydroxyapatite and/or amorphous calcium phosphate obtained in step (b), and

    (d) cooling the sample obtained in step (c) maintaining the DC voltage or the equivalent electric field or

    cooling the sample obtained in step (c) maintaining or without maintaining the electrostatic discharge or the equivalent electric field,
    wherein
    for performing step (c), the sintered sample of hydroxyapatite and/or amorphous calcium phosphate obtained in step (b) or the shaped body obtained from the sintered sample of hydroxyapatite and/or amorphous calcium phosphate obtained in step (b) is arranged between a positive electrode and a negative electrode, which are used for applying the constant or variable DC voltage, equivalent electric field or electrostatic discharge during step (c), such that the sintered sample of hydroxyapatite and/or amorphous calcium phosphate obtained in step (b) or the shaped body obtained from the sintered sample of hydroxyapatite and/or amorphous calcium phosphate obtained in step (b) is spaced from the positive electrode.

**Patentansprüche**

1.  Zusammensetzung oder Material, umfassend

    - permanent polarisierten Hydroxyapatit, wobei das entsprechende $^{31}$P-NMR-Spektrum des permanent polarisierten Hydroxyapatits mit festem Hydroxyapatit unter Verwendung von Phosphorsäure als Referenz bestimmt wird und ein eindeutiges Maximum bei 2,6 ppm oder etwa 2,6 ppm, d.h. in dem Bereich von 2,5 ppm bis 2,7 ppm, aufweist, und
    - Brushit, wobei in dem Weitwinkel-Röntgenstreuspektrum die markantesten Maxima von Brushit bei 2θ = 29°,

EP 4 000 728 B1

31°, 35°, 42° und 51° erscheinen, und/oder ein Brushit-artiges Material, wobei das Brushit-artige Material in dem Raman-Spektrum Maxima bei 878, 848 und 794 cm$^{-1}$ zeigt, und wobei der permanent polarisierte Hydroxyapatit einen Anteil von 50 Gew.-% bis 99,9 Gew.-%, bezogen auf das Gesamtgewicht der Zusammensetzung oder des Materials, aufweist.

2. Zusammensetzung oder Material gemäß Anspruch 1, **dadurch gekennzeichnet, dass** die Zusammensetzung oder das Material in der Form eines Katalysators, vorzugsweise eines mehrphasigen Katalysators, vorliegt, wobei der permanent polarisierte Hydroxyapatit eine Phase, insbesondere eine Hauptphase, des Katalysators bildet und der Brushit und/oder das Brushit-artige Material eine weitere Phase des Katalysators bildet.

3. Zusammensetzung oder Material gemäß Anspruch 1 oder 2, **dadurch gekennzeichnet, dass** die Zusammensetzung oder das Material ein wie in Figur 1(a) gezeigtes Weitwinkel-Röntgenstreumuster aufweist.

4. Zusammensetzung oder Material gemäß einem der vorstehenden Ansprüche, **dadurch gekennzeichnet, dass** die Zusammensetzung oder das Material ein wie in Figur 1(b) gezeigtes Raman-Spektrum aufweist.

5. Zusammensetzung oder Material gemäß einem der vorstehenden Ansprüche, **dadurch gekennzeichnet, dass** der permanent polarisierte Hydroxyapatit einen Anteil von 75 Gew.-% bis 99 Gew.-%, vorzugsweise 80 Gew.-% bis 95 Gew.-%, bezogen auf das Gesamtgewicht der Zusammensetzung oder des Materials, aufweist.

6. Zusammensetzung oder Material gemäß einem der vorstehenden Ansprüche, **dadurch gekennzeichnet, dass** der Brushit und/oder das Brushit-artige Material einen Anteil von 0,1 Gew.-% bis 35 Gew.-%, insbesondere 1 Gew.-% bis 25 Gew.-%, vorzugsweise 5 Gew.-% bis 20 Gew.-%, bezogen auf das Gesamtgewicht der Zusammensetzung oder des Materials, aufweist.

7. Zusammensetzung oder Material gemäß einem der vorstehenden Ansprüche, **dadurch gekennzeichnet, dass** der Brushit und/oder das Brushit-artige Material eine Kristallinität, bestimmt durch Weitwinkel-Röntgenstreuung, von 65 % bis 99,9 %, vorzugsweise 75 % bis 99 %, bevorzugter 80 % bis 95 %, aufweist.

8. Zusammensetzung oder Material gemäß einem der vorstehenden Ansprüche, **dadurch gekennzeichnet, dass** der Brushit und/oder das Brushit-artige Material eine Kristallitgröße, bestimmt durch Weitwinkel-Röntgenstreuung, von 20 nm bis 500 nm, insbesondere 50 nm bis 200 nm, vorzugsweise 70 nm bis 100 nm, aufweist.

9. Verfahren zur Herstellung einer Zusammensetzung oder eines Materials gemäß einem der vorstehenden Ansprüche, **dadurch gekennzeichnet, dass** das Verfahren die folgenden Schritte umfasst:

(a) Bereitstellen einer Probe von Hydroxyapatit und/oder amorphem Calciumphosphat,
(b) Sintern der bei Schritt (a) bereitgestellten Probe von Hydroxyapatit und/oder amorphem Calciumphosphat,
(c) Anlegen einer konstanten oder variablen DC-Spannung zwischen 250 V und 2500 V, insbesondere für wenigstens 1 Minute und/oder bei einer Temperatur zwischen 900 °C und 1200 °C, insbesondere von 1000 °C bis 1200 °C, an die bei Schritt (b) erhaltene gesinterte Probe von Hydroxyapatit und/oder amorphem Calciumphosphat oder an einen aus der bei Schritt (b) erhaltenen gesinterten Probe von Hydroxyapatit und/oder amorphem Calciumphosphat erhaltenen Formkörper, oder

Anlegen eines äquivalenten elektrischen Felds zwischen 1,49 kV/cm und 15 kV/cm, insbesondere für wenigstens 1 Minute und/oder bei einer Temperatur zwischen 900 °C und 1200 °C, insbesondere von 1000 °C bis 1200 °C, an die bei Schritt (b) erhaltene gesinterte Probe von Hydroxyapatit und/oder amorphem Calciumphosphat oder an einen aus der bei Schritt (b) erhaltenen gesinterten Probe von Hydroxyapatit und/oder amorphem Calciumphosphat erhaltenen Formkörper, oder

Anlegen einer elektrostatischen Entladung zwischen 2500 V und 1500000 V, insbesondere für > 0 min bis 24 h und/oder bei einer Temperatur zwischen 900 °C und 1200 °C, insbesondere von 1000 °C bis 1200 °C, an die bei Schritt (b) erhaltene gesinterte Probe von Hydroxyapatit und/oder amorphem Calciumphosphat oder an einen aus der bei Schritt (b) erhaltenen gesinterten Probe von Hydroxyapatit und/oder amorphem Calciumphosphat erhaltenen Formkörper, oder

Anlegen eines äquivalenten elektrischen Felds zwischen 148,9 kV/cm und 8928 kV/cm, insbesondere für > 0 min bis 24 h und/oder bei einer Temperatur zwischen 900 °C und 1200 °C, insbesondere von 1000 °C bis 1200 °C, an die bei Schritt (b) erhaltene gesinterte Probe von Hydroxyapatit und/oder amorphem Calciumphosphat oder an einen aus der bei Schritt (b) erhaltenen gesinterten Probe von Hydroxyapatit und/oder

22

amorphem Calciumphosphat erhaltenen Formkörper, und

(d) Kühlen der bei Schritt (c) erhaltenen Probe unter Halten der DC-Spannung oder des äquivalenten elektrischen Felds, oder

Kühlen der bei Schritt (c) erhaltenen Probe mit oder ohne Halten der elektrostatischen Entladung oder des äquivalenten elektrischen Felds,
wobei
zum Durchführen von Schritt (c) die bei Schritt (b) erhaltene gesinterte Probe von Hydroxyapatit und/oder amorphem Calciumphosphat oder der aus der bei Schritt (b) erhaltenen gesinterten Probe von Hydroxyapatit und/oder amorphem Calciumphosphat erhaltene Formkörper zwischen einer positiven Elektrode und einer negativen Elektrode angeordnet wird, die zum Anlegen der konstanten oder variablen DC-Spannung, des äquivalenten elektrischen Felds oder der elektrostatischen Entladung bei Schritt (c) verwendet werden, wobei die bei Schritt (b) erhaltene gesinterte Probe von Hydroxyapatit und/oder amorphem Calciumphosphat oder der aus der bei Schritt (b) erhaltenen gesinterten Probe von Hydroxyapatit und/oder amorphem Calciumphosphat erhaltene Formkörper von der positiven Elektrode beabstandet ist.

**Revendications**

1. Composition ou matériau comprenant

    - une hydroxyapatite polarisée de manière permanente, le spectre de RMN du ³¹P correspondant de l'hydroxyapatite polarisée de manière permanente étant réalisé avec une hydroxyapatite solide en utilisant de l'acide phosphorique en tant que référence et présentant un pic unique à 2,6 ppm ou à environ 2,6 ppm, c'est-à-dire dans la plage de 2,5 ppm à 2,7 ppm, et
    - une brushite, dans le spectre de diffusion des rayons X aux grands angles, les pics les plus représentatifs de la brushite apparaissant à $2\theta = 29°$, $31°$, $35°$, $42°$ et $51°$ et/ou un matériau de type brushite, le matériau de type brushite présentant dans le spectre Raman des pics à 878, 848 et 794 cm⁻¹, et l'hydroxyapatite polarisée de manière permanente possédant une proportion de 50 % en poids à 99,9 % en poids, sur la base du poids total de la composition ou du matériau.

2. Composition ou matériau selon la revendication 1, caractérisé(e) en ce que la composition ou le matériau est sous la forme d'un catalyseur, préférablement d'un catalyseur à plusieurs phases, l'hydroxyapatite polarisée de manière permanente formant une phase, en particulier une phase principale, du catalyseur et la brushite et/ou le matériau de type brushite formant une phase supplémentaire du catalyseur.

3. Composition ou matériau selon la revendication 1 ou 2, caractérisé (e) en ce que la composition ou le matériau possède un diagramme de diffusion des rayons X aux grands angles tel que présenté sur la figure 1(a).

4. Composition ou matériau selon l'une quelconque des revendications précédentes, caractérisé(e) en ce que la composition ou le matériau possède un spectre Raman tel que présenté sur la figure 1(b).

5. Composition ou matériau selon l'une quelconque des revendications précédentes, caractérisé(e) en ce que l'hydroxyapatite polarisée de manière permanente possède une proportion de 75 % en poids à 99 % en poids, préférablement de 80 % en poids à 95 % en poids, sur la base du poids total de la composition ou du matériau.

6. Composition ou matériau selon l'une quelconque des revendications précédentes, caractérisé(e) en ce que la brushite et/ou le matériau de type brushite possède une proportion de 0,1 % en poids à 35 % en poids, en particulier de 1 % en poids à 25 % en poids, préférablement de 5 % en poids à 20 % en poids, sur la base du poids total de la composition ou du matériau.

7. Composition ou matériau selon l'une quelconque des revendications précédentes, caractérisé(e) en ce que la brushite et/ou le matériau de type brushite possède une cristallinité, déterminée via diffusion de rayons X aux grands angles, de 65 % à 99,9 %, préférablement de 75 % à 99 %, plus préférablement de 80 % à 95 %.

8. Composition ou matériau selon l'une quelconque des revendications précédentes, caractérisé(e) en ce que la brushite et/ou le matériau de type brushite possède une taille de cristallite, déterminée via une diffusion de rayons X aux grands angles, de 20 nm à 500 nm, en particulier de 50 nm à 200 nm, préférablement de 70 nm à 100 nm.

9. Procédé pour la production d'une composition ou d'un matériau selon l'une quelconque des revendications précédentes, **caractérisé en ce que** le procédé comprend les étapes suivantes :

(a) mise à disposition d'un échantillon d'hydroxyapatite et/ou de phosphate de calcium amorphe,

(b) frittage de l'échantillon d'hydroxyapatite et/ou de phosphate de calcium amorphe mis à disposition dans l'étape (a),

(c) application d'une tension CC constante ou variable comprise entre 250 V et 2 500 V, en particulier pendant 1 minute et/ou à une température comprise entre 900 °C et 1 200 °C, en particulier de 1 000 °C à 1 200 °C, à l'échantillon fritté d'hydroxyapatite et/ou de phosphate de calcium amorphe obtenu dans l'étape (b) ou à un corps façonné obtenu à partir de l'échantillon fritté d'hydroxyapatite et/ou de phosphate de calcium amorphe obtenu dans l'étape (b) ou

application d'un champ électrique équivalent compris entre 1,49 kV/cm et 15 kV/cm, en particulier pendant 1 minute et/ou à une température comprise entre 900 °C et 1 200 °C, en particulier de 1 000 °C à 1 200 °C, à l'échantillon fritté d'hydroxyapatite et/ou de phosphate de calcium amorphe obtenu dans l'étape (b) ou à un corps façonné obtenu à partir de l'échantillon fritté d'hydroxyapatite et/ou de phosphate de calcium amorphe obtenu dans l'étape (b) ou

application d'une décharge électrostatique comprise entre 2 500 V et 1 500 000 V, en particulier pendant > 0 minute à 24 h et/ou à une température comprise entre 900 °C et 1 200 °C, en particulier de 1 000 °C à 1 200 °C, à l'échantillon fritté d'hydroxyapatite et/ou de phosphate de calcium amorphe obtenu dans l'étape (b) ou à un corps façonné obtenu à partir de l'échantillon fritté d'hydroxyapatite et/ou de phosphate de calcium amorphe obtenu dans l'étape (b) ou

application d'un champ électrique équivalent compris entre 148,9 kV/cm et 8 928 kV/cm, en particulier pendant > 0 minute à 24 h et/ou à une température comprise entre 900 °C et 1 200 °C, en particulier de 1 000 °C à 1 200 °C, à l'échantillon fritté d'hydroxyapatite et/ou de phosphate de calcium amorphe obtenu dans l'étape (b) ou à un corps façonné obtenu à partir de l'échantillon fritté d'hydroxyapatite et/ou de phosphate de calcium amorphe obtenu dans l'étape (b), et

(d) refroidissement de l'échantillon obtenu dans l'étape (c) en maintenant la tension CC ou le champ électrique équivalent ou

refroidissement de l'échantillon obtenu dans l'étape (c) en maintenant ou sans maintenir la décharge électrostatique ou le champ électrique équivalent,
dans lequel
pour la réalisation de l'étape (c), l'échantillon fritté d'hydroxyapatite et/ou de phosphate de calcium amorphe obtenu dans l'étape (b) ou le corps façonné obtenu de l'échantillon fritté d'hydroxyapatite et/ou de phosphate de calcium amorphe obtenu dans l'étape (b) est agencé entre une électrode positive et une électrode négative, qui sont utilisées pour appliquer la tension CC constante ou variable, le champ électrique équivalent ou la décharge électrostatique pendant l'étape (c), de sorte que l'échantillon fritté d'hydroxyapatite et/ou de phosphate de calcium amorphe obtenu dans l'étape (b) ou le corps façonné obtenu à partir de l'échantillon fritté d'hydroxyapatite et/ou de phosphate de calcium amorphe obtenu dans l'étape (b) soit espacé de l'électrode positive.

Fig. 1 (a)

Fig. 1 (b)

Fig. 1 (c)

Fig. 1 (d)

Fig. 2

Fig. 3

Fig. 4

Fig. 5

Fig. 6

Fig. 7(a)

Fig. 7(b)

Fig. 8(a)

Fig. 8(b)

Fig. 9(a)

Fig. 9(b)

Fig. 10

Fig. 11

Fig. 12

Fig. 13

Fig. 14

Fig. 15

Fig. 16

Fig. 17

Fig. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3278828 A

- WO 2018024727 A1

**Non-patent literature cited in the description**

- **ITOH, S ; NAKAMURA, S ; KOBAYASHI, T ; SHI- NOMIYA, K ; YAMASHITA, K. ; ITOH, S.** Effect of Electrical Polarization of Hydroxyapatite Ceramics on New Bone Formation. *Calcif. Tissue Int.,* 2006, vol. 78, 133-142
- **NAKAMURA, S ; KOBAYASHI, T. ; YAMASHITA, K.** Highly Orientated Calcification in Newly Formed Bones on Negatively Charged Hydroxyapatite Electrets. *Key Eng. Mater.,* 2005, 284-286, 897-900

- **RIVAS, M. ; DEL VALLE, L. J. ; ARMELIN, E ; BER- TRAN, O. ; TURON, P. ; PUIGGALI, J. ; ALEMÁN, C.** Hydroxyapatite with Permanent Electrical Polarization: Preparation, Characterization, and Response against Inorganic Adsorbates. *Chem. Phys. Chem.,* 2018, vol. 19, 1746-1755
- **RIVAS, M. ; DEL VALLE, L. J. ; TURON, P ; ALE- MÁN, C. ; PUIGGALI, J.** Sustainable Synthesis of Amino Acids by Catalytic Fixation of Molecular Dinitrogen and Carbon Dioxide. *Green Chem.,* 2018, vol. 20, 685-693